# EUROPÄISCHE PATENTSCHRIFT

(11) **EP 3 645 598 B1**
(45) Veröffentlichungstag und Bekanntmachung des Hinweises auf die Patenterteilung: **19.06.2024**
(21) Anmeldenummer: 18733881.9
(22) Anmeldetag: 29.06.2018
(51) Int. Cl.: C08G 18/67, C08G 18/81, C08G 18/24, G03F 7/00, B33Y 70/00, B29C 64/124, B32B 27/40, C07C 269/02, C08G 18/73, C08G 18/79

(54) **ADDITIVES HERSTELLUNGSVERFAHREN MIT EINEM THERMOPLASTISCHEN RADIKALISCH VERNETZBAREN AUFBAUMATERIAL**
ADDITIVE MANUFACTURING METHOD COMPRISING A THERMOPLASTIC STRUCTURAL MATERIAL WHICH IS CROSSLINKABLE WITH RADICALS
PROCÉDÉ DE FABRICATION ADDITIVE AU MOYEN DE MATÉRIAU DE CONSTRUCTION THERMOPLASTIQUE RÉTICULÉ DE FAÇON RADICALAIRE

(30) Priorität: 30.06.2017 EP 17179206
(43) Veröffentlichungstag der Anmeldung: 06.05.2020
(73) Patentinhaber: Stratasys, Inc., Eden Prairie, MN 55344 (US)
(72) Erfinder: ACHTEN, Dirk, 51375 Leverkusen (DE); BÜSGEN, Thomas, 51377 Leverkusen (DE); TOMCZYK, Christoph, 51379 Leverkusen (DE); LUDEWIG, Michael, 51519 Odenthal (DE); FÄCKE, Thomas, 51375 Leverkusen (DE); WAGNER, Roland, 51375 Leverkusen (DE); STEMPFLE, Florian, Johannes, 50825 Köln (DE)
(74) Vertreter: Davepon, Björn
(86) Internationale Anmeldenummer: PCT/EP2018/067557
(87) Internationale Veröffentlichungsnummer: WO 2019/002540

(56) Entgegenhaltungen:
- EP-A1- 2 526 137
- WO-A1-2012/004088
- WO-A1-2015/200189
- WO-A1-2017/112653

## Beschreibung

Beschichtungsmittel, die durch zwei unabhängige Prozesse aushärten, werden allgemein als Dual Cure-Systeme bezeichnet. Üblicherweise besitzen die enthaltenen Bindemittelkomponenten dabei unterschiedliche funktionelle Gruppen die unter geeigneten Bedingungen in der Regel unabhängig voneinander miteinander vernetzen. Übliche zum Stand der Technik gehörende Dual Cure-Systeme besitzen strahlen- sowie thermisch härtbare Gruppen, wobei besonders vorteilhafte Eigenschaften bei Verwendung von Isocyanat- und Hydroxygruppen als thermisch vernetzende Funktionen erhalten werden. Nachteilig an solchen Lösungen ist jedoch, dass die Reaktivität der NCO Gruppen und/oder die Anwesenheit von Katalysatoren für den zweiten Härtungsmechnismus die Topfzeit des Beschichtungsmittels begrenzt.

Eine Klasse von Dual Cure-Systemen enthält blockierte Isocyanate. Nach einer Deblockierung bei geeigneter Temperatur stehen die NCO-Gruppen für Reaktionen mit Polyolen zur Verfügung. Nachteilig bei der Verwendung von blockierten Isocyanaten sind die für blockierte Isocyanate typische hohe Viskosität und die üblicherweise sehr hohe Deblockierungstemperatur.

Dual Cure-Systeme können in Beschichtungsanwendungen und bei Verwendung als Klebstoffe Vorteile bei der sogenannten Schattenhärtung aufweisen. Hierunter ist derjenige Härtungsmechanismus zu verstehen, welcher nicht photochemisch, sondern beispielsweise thermisch abläuft. Dann kann das Beschichtungs- oder Klebmittel auch bei komplex geformten Substraten mit Abschattungen gegenüber einer Belichtungslampe weiter aushärten.

Im Lack- und Klebstoffbereich existieren mehrere Hauptgruppen der Dual Cure-Technologie: zwei verschiedene Radikalstarter (UV und thermisch), UV- und Feuchtigkeitsnachhärtung, UV- und PUR-2K-Härtung und eine kationisch katalysierte UV- und thermische Härtung. Von der Firma Berlac AG wird beispielsweise unter der Bezeichnung Berlac 082.907 ein Dual Cure-Lacksystem angeboten, in dem zunächst eine Reaktion zwischen NCO-Gruppen und OH-Gruppen ausgelöst wird und dann einer UV-Härtung unterworfen wird.

Eine weitere denkbare Anwendung von Dual Cure-Systemen ist in additiven Fertigungsverfahren ("3D-Druck"). Als additive Fertigungsverfahren werden solche Verfahren bezeichnet, mit denen Gegenstände schichtweise aufgebaut werden. Sie unterscheiden sich daher deutlich von anderen Verfahren zur Herstellung von Gegenständen wie Fräsen oder Bohren. Bei letztgenannten Verfahren wird ein Gegenstand so bearbeitet, dass er durch Wegnahme von Material seine Endgeometrie erhält.

Additive Fertigungsverfahren nutzen unterschiedliche Materialien und Prozesstechniken, um Gegenstände schichtweise aufzubauen. Eine Gruppe von additiven Fertigungsverfahren setzt radikalisch vernetzbare Harze ein, welche gegebenenfalls über einen zweiten Härtungsmechanismus ihre Endfestigkeit im gebildeten Gegenstand erhalten. Beispiele für solche Verfahren sind Stereolithographieverfahren und das davon abgeleitete sogenannte DLP-Verfahren.

Für den 3D-Druck bedeuten die Nachteile von konventionellen Dual Cure-Systemen hinsichtlich der Topfzeit, dass ein nicht gebrauchtes Aufbaumaterial schlecht wiederverwendet werden kann bzw. die geplanten Bauzeiten für ein Produkt die Topfzeit nicht überschreiten dürfen.

Wünschenswert wären daher 3D-Druckverfahren, in denen die genannten Nachteile nicht auftreten.

WO 2016/181402 A1 offenbart ein Verfahren zur Herstellung eines polymeren dreidimensionalen Objekts durch Drucken eines photopolymerisierbaren Materials in der Schmelze in ein Objekt mit einer speicherbaren ersten Form, welche zu einer metastabilen Form deformierbar ist und welche oberhalb einer Triggertemperatur des Polymers in die erste Form übergehen kann. In einem beschriebenen Experiment wird ein Polycaprolacton mit Isocyanatoethylmethacrylat umgesetzt und das erhaltene Makromonomer bei 90 °C als Schmelze in einem 3D-Drucker unter Einsatz von UV-Licht (385 nm) zu einem Gegenstand verarbeitet.

Weitere Dokumente aus dem Stand der Technik sind WO 2016/181149 A1 ("Method for making an object"), WO 2015/200189 A1 ("Three-dimensional objects produced from materials having multiple mechanisms of hardening"), WO 2016/039986 A1 ("Build materials having a metallic appearance for 3D printing") und US 2001/0025061 A1 ("Thermally-stable photopolymer composition and light transmissive device").

Eine Aufgabe der vorliegenden Erfindung ist es, mindestens einen Nachteil des Standes der Technik wenigstens zu einem Teil zu überwinden. Weiterhin stellt sich die Erfindung die Aufgabe, ein additives Fertigungsverfahren bereitzustellen, bei dem die herzustellenden Gegenstände aus einem Aufbaumaterial mit mehr als einem Mechanismus der Erhöhung der mechanischen Festigkeit möglichst kosteneffizient und/oder individualisiert und/oder ressourcenschonend erhalten werden können, was insbesondere die Wiederverwertbarkeit von Aufbaumaterial betrifft.

Erfindungsgemäß gelöst wird die Aufgabe durch ein Verfahren gemäß Anspruch 1, ein System gemäß Anspruch 11 und eine Verwendung gemäß Anspruch 13. Vorteilhafte Weiterbildungen sind in den Unteransprüchen angegeben. Sie können beliebig kombiniert werden, sofern sich aus dem Kontext nicht eindeutig das Gegenteil ergibt.

Ein Verfahren zur Herstellung eines Gegenstandes in einem additiven Fertigungsverfahren aus einem Vorläufer umfasst die Schritte:
I) Abscheiden einer Lage eines radikalisch vernetzten Aufbaumaterials, welche einem ersten ausgewählten Querschnitt des Vorläufers entspricht, auf einem Träger;
II) Abscheiden einer Lage eines radikalisch vernetzten Aufbaumaterials, welche einem weiteren ausgewählten Querschnitt des Vorläufers entspricht, auf eine zuvor aufgetragene Lage des radikalisch vernetzten Aufbaumaterials;
III) Wiederholen des Schritts II), bis der Vorläufer gebildet ist;

wobei das Abscheiden eines radikalisch vernetzten Aufbaumaterials wenigstens in Schritt II) durch Belichten und/oder Bestrahlen eines ausgewählten Bereichs eines radikalisch vernetzbaren Aufbaumaterials, entsprechend dem jeweils ausgewählten Querschnitt des Vorläufers, erfolgt;
wobei der Träger innerhalb eines Behälters angeordnet ist, der Behälter das radikalisch vernetzbare Aufbaumaterial bereitstellt, der Träger vertikal entgegen der Schwerkraftrichtung anhebbar ist und weiterhin vor jedem Schritt II) der Träger um eine vorbestimmte Strecke angehoben wird, so dass unter der in vertikaler Richtung gesehen untersten Lage des Aufbaumaterials sich eine Schicht des radikalisch vernetzbaren Aufbaumaterials bildet.

Das radikalisch vernetzbare Aufbaumaterial umfasst ein thermoplastisches radikalisch vernetzbares Polyurethan mit einem Urethangruppengehalt von ≥ 5 Gewichts-% und einen Photoinitiator und wird in Schritt II) auf eine Verarbeitungstemperatur erwärmt, welche größer als der Schmelzpunkt, bestimmt mittels dynamischer Differenzkalorimetrie, erste Aufheizung, bei einer Heizrate von 20 K/min, des radikalisch vernetzbaren Polyurethans ist.

Nach Schritt III) wird der eine Temperatur von 20 °C aufweisende Vorläufer als der Gegenstand definiert oder nach Schritt III) wird der Schritt IV) durchgeführt:
IV) Durchführen einer chemischen Reaktion im nach Schritt III) erhaltenen Vorläufer, so dass der Gegenstand erhalten wird.

Im erfindungsgemäßen Verfahren wird somit der Gegenstand in mindestens zwei Herstellungsabschnitten erhalten. Der erste Herstellungsabschnitt kann als Aufbauabschnitt angesehen werden. Dieser Aufbauabschnitt lässt sich mittels strahlenoptischer additiver Fertigungsverfahren wie dem DLP (digital light processing)-Verfahren realisieren und ist Gegenstand der Schritte I), II) und III). Der zweite Herstellungsabschnitt kann als

Härtungsabschnitt angesehen werden. Hier wird der nach dem Aufbauabschnitt erhaltene Vorläufer oder intermediäre Gegenstand ohne seine Form weiter zu verändern in einen mechanisch dauerhafteren Gegenstand überführt. Das Material, aus dem der Vorläufer im additiven Fertigungsverfahren erhalten wird, wird im Rahmen der vorliegenden Erfindung allgemein als "Aufbaumaterial" bezeichnet.

In Schritt I) des Verfahrens erfolgt das Abscheiden eines radikalisch vernetzten Aufbaumaterials auf einem Träger. Dieses ist gewöhnlich der erste Schritt in DLP-Verfahren. Auf diese Weise wird eine Lage eines mit dem Träger verbundenen Aufbaumaterials erhalten, welche einem ersten ausgewählten Querschnitt des Vorläufers entspricht.

Gemäß der Anweisung von Schritt III) wird Schritt II) so lange wiederholt, bis der gewünschte Vorläufer gebildet ist. In Schritt II) erfolgt das Abscheiden eines radikalisch vernetzten Aufbaumaterials auf eine zuvor aufgetragene Lage des Aufbaumaterials, so dass eine weitere Lage des Aufbaumaterials erhalten wird, welche einem weiteren ausgewählten Querschnitt des Vorläufers entspricht und welche mit der zuvor aufgetragenen Lage verbunden ist. Bei der zuvor aufgetragenen Lage des Aufbaumaterials kann es sich um die erste Lage aus Schritt I) oder um eine Lage aus einer vorigen Durchlauf des Schritts II) handeln.

Es ist erfindungsgemäß vorgesehen, dass das Abscheiden eines radikalisch vernetzten Aufbaumaterials wenigstens in Schritt II) (vorzugsweise auch in Schritt I) durch Belichten und/oder Bestrahlen eines ausgewählten Bereichs eines radikalisch vernetzbaren Aufbaumaterials, entsprechend dem jeweils ausgewählten Querschnitt des Gegenstandes, erfolgt. Im Kontext der vorliegenden Erfindung werden die Begriffe "radikalisch vernetzbares Aufbaumaterial" und "radikalisch vernetztes Aufbaumaterial" benutzt. Hierbei wird das radikalisch vernetzbare Aufbaumaterial durch das Belichten und/oder Bestrahlen, welches radikalische Vernetzungsreaktionen auslöst, in das radikalisch vernetzte Aufbaumaterial überführt. Unter "Belichten" wird hierbei die Einwirkung von Licht im Bereich zwischen nahem IR- und nahem UV-Licht (1400 nm bis 315 nm Wellenlänge) verstanden. Die übrigen kürzeren Wellenlängenbereiche werden durch den Begriff "Bestrahlen" abgedeckt, zum Beispiel fernes UV-Licht, Röntgenstrahlung, Gammastrahlung und auch Elektronenstrahlung.

Das Auswählen des jeweiligen Querschnitts erfolgt zweckmäßigerweise durch ein CAD-Programm, mit dem ein Modell des herzustellenden Gegenstandes erzeugt wurde. Diese Operation wird auch "Slicing" genannt, und dient als Grundlage für die Steuerung der Belichtung und/oder Bestrahlung des radikalisch vernetzbaren Aufbaumaterials.

Im Verfahren umfasst das radikalisch vernetzbare Aufbaumaterial ein thermoplastisches radikalisch vernetzbares Polyurethan mit einem Urethangruppengehalt von ≥ 5 Gewichts-% und einen Photoinitiator. Der Urethangruppengehalt lässt sich in Kenntnis des NCO-Gehalts und der OH-Zahl der zur Herstellung des Polyurethans eingesetzten Edukte in bekannter Weise berechnen. Vorzugsweise beträgt der Urethangruppengehalt ≥ 5 Gewichts-% bis ≤ 50 Gewichts-%, bezogen auf das Gesamtgewicht des Polyurethans. Mehr bevorzugt beträgt er ≥ 10 Gewichts-% bis ≤ 40 Gewichts-%, besonders bevorzugt ≥ 15 Gewichts-% bis ≤ 30 Gewichts-%.

Das radikalisch vernetzbare Polyurethan lässt sich beispielsweise aus der Reaktion eines NCO-terminierten Polyurethans mit einem Hydroxyalkyl(meth)acrylat erhalten. Ein weiterer Zugang besteht in der Reaktion eines OH-terminierten Polyurethans mit NCO-terminierten (Meth)acrylaten wie Isocyanatoethyl(meth)acrylat.

Geeignete Diisocyanate zur Herstellung der Polyurethane sind beispielsweise solche, die ein Molekulargewicht im Bereich von 140 bis 400 g/mol aufweisen, mit aliphatisch, cycloaliphatisch, araliphatisch und/oder aromatisch gebundenen Isocyanatgruppen, wie z. B. 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethyl-cyclohexan (Isophorondiisocyanat; IPDI), 1-Isocyanato-1-methyl-4(3)-isocyanatomethylcyclohexan, 2,4'- und 4,4'-Diisocyanatodicyclohexylmethan (H₁₂MDI), 1,3- und 1,4-Bis(isocyanatomethyl)cyclohexan, Bis-(isocyanatomethyl)-norbornan (NBDI), 4,4'-Diisocyanato-3,3'-dimethyldicyclohexylmethan, 4,4'-Diisocyanato-3,3',5,5'-tetramethyldicyclohexylmethan, 4,4'-Diisocyanato-1,1'-bi(cyclohexyl), 4,4'-Diisocyanato-3,3'-dimethyl-1,1-bi(cyclohexyl), 4,4'-Diisocyanato-2,2',5,5'-tetra-methyl-1,1'-bi(cyclohexyl), 1,8-Diisocyanato-p-menthan, 1,3-Diisocyanato-adamantan, 1,3-Dimethyl-5,7-diisocyanatoadamantan, 1,3- und 1,4-Bis-(isocyanatomethyl)benzol (Xyxlylendiisocyanat; XDI), 1,3- und 1,4-Bis(1-isocyanato-1-methylethyl)-benzol (TMXDI) und Bis(4-(1-isocyanato-1-methylethyl)phenyl)-carbonat, 2,4- und 2,6-Diisocyanatotoluol (TDI), 2,4'- und 4,4'-Diisocyanatodiphenylmethan (MDI), 1,5-Diisocyanatonaphthalin sowie beliebige Gemische solcher Diisocyanate.

Beispiele für geeignete Polyole sind bevorzugt lineare Polyether-, Polyester-, Polyacrylat, Polyepoxyd oder Polycarbonat-Polyole. Ferner können erfindungsgemäß auch Prepolymere, wie beispielsweise Polyether-, Polyester-, Polyacrylat, Polyepoxyd oder Polycarbonat-Prepolymere als Edukte der Polyurethan-Bildung eingesetzt werden.

Geeignete Hydroxyalkyl(meth)acrylate sind unter anderem Alkoxyalkyl(meth)acrylate mit 2 bis 12 Kohlenstoffatomen im Hydroxyalkylrest. Bevorzugt sind 2-Hydroxyethylacrylat, das bei der Anlagerung von Propylenoxid an Acrylsäure entstehende Isomerengemisch oder 4-Hydroxybutylacrylat, sowie die analogen Methacrylate.

Im Verfahren wird in Schritt II) das radikalisch vernetzbare Aufbaumaterial auf eine Verarbeitungstemperatur erwärmt, welche größer als der Schmelzpunkt des radikalisch vernetzbaren Polyurethans ist. Der Schmelzpunkt wird zweckmäßigerweise mittels DSC (differential scanning calorimetry) bestimmt. Die Temperatur kann ≥ 2 °C bis ≤ 100 °C, vorzugsweise ≥ 5 °C bis ≤ 80 °C und mehr bevorzugt ≥ 10 °C bis ≤ 60 °C über dem mittels DSC ermittelten Schmelzpunkt des radikalisch vernetzbaren Polyurethans liegen.

Bevorzugt liegt die Verarbeitungstemperatur des Aufbaumaterials oberhalb der Raumtemperatur (RT) von 23 °C. Bevorzugt liegt die Verarbeitungstemperatur des Aufbaumaterials mindestens 5 °C, oder bevorzugt mindestens 10 °C, oder bevorzugt mindestens 25 °C, oder bevorzugt mindestens 50 °C oberhalb der Raumtemperatur (RT) von 23 °C.

Es ist möglich und vorteilhaft, wenn das erfindungsgemäße Verfahren so durchgeführt wird, dass wenigstens ein Teil der in zuvor durchgeführten Schritten II) abgeschiedenen Lagen des radikalisch vernetzten Aufbaumaterials auf einer Temperatur oberhalb ihres Schmelzpunktes gehalten werden, wobei das Material in der Schmelze vernetzt ist, das heißt der Gelpunkt überschritten wurde. Hierbei kann es sich beispielsweise um die letzten 100, bevorzugt die letzten 50 oder besonders bevorzugt die letzten 15 Lagen handeln. Der Gelpunkt wird als erreicht angesehen, wenn in einer dynamisch-mechanischen Analyse (DMA) mit einem Platte/Platte-Oszillationsviskosimeter gemäß ISO 6721-10 bei 20 °C sich die Graphen des Speichermoduls G` und des Verlustmoduls G" kreuzen. Bei dieser Vorgehensweise können mechanische Spannungen durch Abkühlen und Kristallisieren während des 3D-Druckvorgangs verringert oder ganz vermieden werden und gleichzeitig eine bessere Haftung der gerade abgeschiedenen Lage zur jeweils zuvor abgeschiedenen Lage erreicht werden.

Diese besondere Ausführungsform ist deshalb bevorzugt, weil so der Vorläufer weitgehend spannungsarm zum Produkt überführt wird und gleichzeitig die unteren Schichten eine gute Haftung zur jeweils nächsten Aufbauschicht ausbilden.

Nach Schritt III) bietet das erfindungsgemäße Verfahren zwei Alternativen. Entweder kann keine weitere chemische Reaktion im erhaltenen Vorläufer durchgeführt werden oder es wird eben solch eine chemische Reaktion durchgeführt. In der ersten Variante wird der Vorläufer, sobald er eine Temperatur von 20 °C eingenommen hat (beispielsweise durch Entnehmen aus dem 3D-Drucker und Abkühlen lassen) als der zu erhaltene Gegenstand definiert. Der Gegenstand hat gegenüber dem Vorläufer eine erhöhte mechanische Festigkeit und das Herstellungsverfahren hinsichtlich dieses Gegenstandes beendet. In der zweiten Variante schließt sich nach Schritt III) der Schritt IV) an, in dem Vorläufer beziehungsweise dessen radikalisch vernetztem Aufbaumaterial eine chemische Reaktion durchgeführt wird. Diese Reaktion kann beispielsweise thermisch initiiert sein. Auch hierdurch erhält der Gegenstand gegenüber dem Vorläufer eine erhöhte mechanische Festigkeit.

Es ist möglich, dass, beispielsweise aufgrund der Temperierung des 3D-Druckers oder einer langsamen Kristallisation des radikalisch vernetzten Aufbaumaterials im nach Schritt III) erhaltenen Vorläufer das Material solange im Zustand einer vernetzten Polymerschmelze verweilt, bis der Vorläufer aus der zu seiner Herstellung verwendeten Vorrichtung entnommen wird.

Es ist bevorzugt, dass Schritt IV) dann durchgeführt wird, wenn das gesamte abgeschiedene Aufbaumaterial des Vorläufers seinen Gelpunkt erreicht hat. Der Gelpunkt wird als erreicht angesehen, wenn in einer dynamisch-mechanischen Analyse (DMA) mit einem Platte/Platte-Oszillationsviskosimeter gemäß ISO 6721-10 bei 20 °C sich die Graphen des Speichermoduls G' und des Verlustmoduls G" kreuzen. Das radikalisch vernetzte Aufbaumaterial kann ein Speichermodul G` (DMA, Platte/Platte-Oszillationsviskosimeter gemäß ISO 6721-10 bei 20 °C und einer Scherrate von 1/s) von ≥ 10⁶ Pa aufweisen.

Das radikalisch vernetzbare Aufbaumaterial kann weiterhin Additive wie Füllstoffe, UV-Stabilisatoren, Radikalinhibitoren, Antioxidantien, Formtrennmittel, Wasserfänger, Slipadditive, Entschäumer, Verlaufsmittel, Rheologieadditive, Flammschutzmittel und/oder Pigmente enthalten. Diese Hilfs- und Zusatzmittel, ausgenommen Füllstoffe und Flammschutzmittel, liegen üblicherweise in einer Menge von weniger als 10 Gew.-%, vorzugsweise weniger als 5 Gew.-%, besonders bevorzugt bis zu 3 Gew.-%, bezogen auf das radikalisch vernetzbare Aufbaumaterial vor. Flammschutzmittel liegen üblicherweise in Mengen von höchstens 70 Gew.-%, vorzugsweise höchstens 50 Gew.-%, besonders bevorzugt höchstens 30 Gew.-%, berechnet als Gesamtmenge an eingesetzten Flammschutzmitteln bezogen auf das Gesamtgewicht des radikalisch vernetzbaren Aufbaumaterials vor.

Geeignete Füllstoffe sind beispielsweise, SiOz, AlOHs, Al₂O₃, CaCO₃, Metallpigmente wie TiO₂ und weitere bekannte übliche Füllstoffe. Diese Füllstoffe werden vorzugsweise in Mengen von höchstens 70 Gew.-%, bevorzugt höchstens 50 Gew.-%, besonders bevorzugt höchstens 30 Gew.-%, berechnet als Gesamtmenge an eingesetzten Füllstoffen bezogen auf das Gesamtgewicht des radikalisch vernetzbaren Aufbaumaterials, eingesetzt.

In einer besonderen Ausführungsform sind die Füllstoffe zumindest teilweise faseriger Natur mit einem Aspektverhältnis von > 10 bis < 5000. Bevorzugte Faserlängen sind > 5 Mikrometer bis < 100 Mikrometer, bevorzugte Faserdichten sind > 0,9 g/cm³ bis < 2 g/cm³, stärker bevorzugt > 0,95 g/cm³ bis < 1,6 g/cm³. Beispielhafte Fasern sind Kohlenstoffasern, PE Fasern, Polyamidfasern, Polyimidfasern, Polyesterfasern, Baumwollfasern, Rayonfasern, Flachsfasern, Jutefasern, Polyurethanfasern. Besonders bevorzugt sind Polyurethanfasern und PE-Fasern.

Geeignete UV-Stabilisatoren können vorzugsweise ausgewählt werden aus der Gruppe, bestehend aus Piperidinderivaten, wie z.B. 4-Benzoyloxy-2,2,6,6-tetramethylpiperidin, 4-Benzoyloxy-1,2,2,6,6-pentamethylpiperidin, Bis-(2,2,6,6-tetra-methyl-4-piperidyl)-sebacat, Bis(1,2,2,6,6-pentamethyl-1-4-piperidinyl)-sebacat, Bis-(2,2,6,6-tetramethyl-4-piperidyl)-suberat, Bis-(2,2,6,6-tetramethyl-4-piperidyl)-dodecandioat; Benzophenonderivaten, wie z.B. 2,4-Dihydroxy-, 2-Hydroxy-4-methoxy-, 2-Hydroxy-4-octoxy-, 2-Hydroxy-4-dodecyloxy- oder 2,2'-Dihydroxy-4-dodecyloxy-benzophenon; Benztriazolderivaten, wie z.B. 2-(2H-Benzotriazol-2-yl)-4,6-di-tert-pentylphenol, 2-(2H-Benzotriazol-2-yl)-6-dodecyl-4-methylphenol, 2-(2H-Benzotriazol-2-yl)-4,6-bis(1-methyl-1-phenylethyl)phenol, 2-(5-Chlor-2H-benzotriazol-2-yl)-6-(1,1-dimethylethyl)-4-methylphenol, 2-(2H-benzotriazol-2-yl)-4-(1,1,3,3-tetramethylbutyl)phenol, 2-(2H-Benzotriazol-2-yl)-6-(1-methyl-1-pbenylethyl)-4-(1,1,3,3-tetramethylbutyl)phenol, Isooctyl-3-(3-(2H-benzotriazol-2-yl)-5-(1,1-dimethylethyl)-4-hydroxyphenylpropionat), 2-(2H-Benzotriazol-2-yl)-4,6-bis(1,1-dimethylethyl)phenol, 2-(2H-Benzotriazol-2-yl)-4,6-bis(1-methyl-1-phenylethyl)phenol, 2-(5-Chlor-2H-benzotriazol-2-yl)-4,6-bis(1,1-dimethylethyl)phenol; Oxalaniliden, wie z.B. 2-Ethyl-2'-ethoxy- oder 4-Methyl-4'-methoxyoxalanilid; Salicylsäureestern, wie z.B. Salicylsäurephenylester, Salicylsäure-4-tert-butylphenylester, Salicylsäure-4-tert-octylphenylester; Zimtsäureesterderivaten, wie z.B. α-Cyano-(3-methyl-4-methoxyzimtsäuremethylester, α-Cyano-(3-methyl-4-methoxyzimtsäurebutyl-ester, α-Cyano-(3-phenylzimtsäureethylestex, α-Cyano-(3-phenylzimtsäureisooctylester; und Malonesterderivaten, wie z.B. 4-Methoxybenzylidenmalonsäuredimethylester, 4-Methoxybenzylidenmalonsäurediethylester, 4-Butoxybenzylidenmalonsäuredimethylester. Diese bevorzugten Lichtstabilisatoren können sowohl einzeln als auch in beliebigen Kombinationen untereinander zum Einsatz kommen.

Besonders bevorzugte UV-Stabilisatoren sind solche, die Strahlung einer Wellenlänge < 400 nm vollständig absorbieren. Hierzu zählen beispielsweise die genannten Benztriazolderivate. Ganz besonders bevorzugte UV-Stabilisatoren sind 2-(5-Chlor-2H-benzotriazol-2-yl)-6-(1,1-dimethylethyl)-4-methylphenol, 2-(2H-benzotriazol-2-yl)-4-(1,1,3,3-tetramethylbutyl)phenol und/oder 2-(5-Chlor-2H-benzotriazol-2-yl)-4,6-bis(1,1-dimethylethyl)phenol.

Gegebenenfalls werden ein oder mehrere der beispielhaft genannten UV-Stabilisatoren dem radikalisch vernetzbaren Aufbaumaterial vorzugsweise in Mengen von 0,001 bis 3,0 Gew.-%, besonders bevorzugt 0,005 bis 2 Gew.-%, berechnet als Gesamtmenge an eingesetzten UV-Stabilisatoren bezogen auf das Gesamtgewicht des radikalisch vernetzbaren Aufbaumaterials, zugesetzt.

Geeignete Antioxidantien sind vorzugsweise sterisch gehinderten Phenole, welche vorzugsweise ausgewählt werden können aus der Gruppe, bestehend aus 2,6-Di-tert-butyl-4-methylphenol (Ionol), Pentaerythrit-tetrakis(3 -(3,5 -di-tert-butyl-4-hydroxy-phenyl)-propionat), Octadecyl-3-(3,5-di-tert-butyl-4-hydroxyphenyl)-propionat, Triethylen-glykol-bis(3-tert-butyl-4-hydroxy-5-methylphenyl)propionat, 2,2'-Thio-bis(4-methyl-6-tert-butylphenol) und 2,2'-Thiodiethyl-bis[3-(3,5-di-tert-butyl-4-hydroxyphenyl)propionat]. Diese können bei Bedarf sowohl einzeln als auch in beliebigen Kombinationen untereinander eingesetzt werden. Diese Antioxidantien werden vorzugsweise in Mengen von 0,01 bis 3,0 Gew.-%, besonders bevorzugt 0,02 bis 2,0 Gew.-%, berechnet als Gesamtmenge an eingesetzten Antioxidantien, bezogen auf das Gesamtgewicht des radikalisch vernetzbaren Aufbaumaterials, eingesetzt.

Geeignete Radikalinhibitoren bzw. Verzögerer sind besonders solche, die eine unkontrollierte radikalische Polymerisation der Formulierung außerhalb des gewünschten (bestrahlten) Bereiches gezielt inhibieren. Diese sind für eine gute Randschärfe und Abbildungsgenauigkeit im Vorläufer entscheidend. Geeignete Radikalinhibitoren müssen entsprechend der gewünschten Radikalausbeute aus dem Bestrahlungs-/Belichtungsschritt und der Polymerisationsgeschwindigkeit und Reaktivität/Selektivität der Doppelbindungsträger ausgesucht werden. Geeignete Radikalinhibitoren sind z. B. 2,2-(2,5-thiophendiyl)bis(5-tertbutylbenzoxazol), Phenothiazin, Hydrochinone, Hydrochinonether, Quinonalkyde und Nitroxylverbindungen sowie Mischungen davon, Benzoquinone, Kupfer Salze, Brenzcatechine, Cresole, Nitrobenzol und Sauerstoff. Diese Antioxidantien werden vorzugsweise in Mengen von 0,001 Gew% bis 3 Gew.-%, bezogen auf das Gesamtgewicht des radikalisch vernetzbaren Aufbaumaterials, eingesetzt.

Ausführungsformen und weitere Aspekte der vorliegenden Erfindung werden im Folgenden erläutert. Sie können beliebig miteinander kombiniert werden, sofern sich aus dem Kontext nicht eindeutig das Gegenteil ergibt.

Bevorzugt weist das radikalisch vernetzbare Polyurethan einen Schmelzpunkt oder Glaspunkt von ≥ 25 °C, oder bevorzugt ≥ 30 °C, oder bevorzugt ≥ 35 °C bis ≤ 150 °C, mehr bevorzugt ≥ 40 °C bis ≤ 120 °C auf (ermittelt mittels DSC).

Bevorzugt weist das radikalisch vernetzbare Polyurethan bei RT eine Viskosität (DIN EN ISO 2884-1) von ≥ 10000 mPas, oder bevorzugt ≥ 20000 mPas, oder bevorzugt ≥ 25000 mPas auf oder ist ein Feststoff.

Das radikalisch vernetzbare Polyurethan weist einen Schmelzpunkt oder Glaspunkt, bestimmt mittels dynamischer Differenzkalorimetrie, erste Aufheizung, bei einer Heizrate von 20 K/min, von ≥ 50 °C, bevorzugt ≥ 60 °C bis ≤ 150 °C, mehr bevorzugt ≥ 80 °C bis ≤ 120 °C auf;
das radikalisch vernetzbare Polyurethan weist bei der Verarbeitungstemperatur vorzugsweise eine Viskosität (DIN EN ISO 2884-1) von ≤ 10000 mPas (bevorzugt ≥ 50 mPas bis ≤ 8000 mPas, mehr bevorzugt ≥ 500 mPas bis ≤ 5000 mPas) auf und/oder
der Photoinitiator bei der Verarbeitungstemperatur weist eine Halbwertszeit für seinen thermischen Abbau von ≥ 1 Stunde, oder bevorzugt ≥ 1,5 Stunden bis ≤ 5 Stunden, mehr bevorzugt ≥ 2 Stunden bis ≤ 4 Stunden auf.

Das Aufbaumaterial weist in Gegenwart des Photoinitiators bei der Verarbeitungstemperatur eine Topfzeit von bevorzugt ≥ 1 Stunde, oder bevorzugt ≥ 1,5 Stunden bis ≤ 5 Stunden, mehr bevorzugt ≥ 2 Stunden bis ≤ 4 Stunden auf. Unter Topfzeit wird im Sinne der Erfindung die Zeit verstanden bei der sich die Viskosität des Aufbauharzes verdoppelt.

In einer weiteren bevorzugten Ausführungsform liegen neben Photoinitiatoren auch thermisch aktivierbare Radikalspender wie z.B. Peroxide vor, die bevorzugt in einem in Schritt IV) durchgeführten Temperprozess bei Temperaturen oberhalb der empfohlenen Zersetzungstemperaturen für diese Radikalspender nicht umgesetzte Doppelbindungen zur Reaktion bringen.

In einer weiteren bevorzugten Ausführungsform liegen in dem radikalisch vernetzbaren Aufbaumaterial weitere, von radikalisch vernetzbaren funktionellen Gruppen verschiedene, funktionelle Gruppen in blockierter oder unblockierter Form vor, welche in der Lage sind, eine von einer radikalischen Vernetzung verschiedene Reaktion zur Erhöhung der mechanischen Festigkeit im Aufbaumaterial einzugehen. Unter einer Reaktion zur Erhöhung der mechanischen Festigkeit wird im Kontext der vorliegenden Erfindung neben einer Vernetzungsreaktion auch jede weitere Reaktion verstanden, durch die sich der Elastizitätsmodul (DIN 53504) des durch das erfindungsgemäße Verfahren erhaltenen Gegenstands bei 20 °C um ≥ 10% (bevorzugt ≥ 20%, mehr bevorzugt ≥ 50%) erhöht. Alternativ oder ergänzend dazu kann eine Erhöhung der Zugfestigkeit (DIN 53504) des durch das erfindungsgemäße Verfahren erhaltenen Gegenstands bei 20 °C um ≥ 10% ( bevorzugt ≥ 20%, mehr bevorzugt ≥ 50%) als Kriterium hierfür herangezogen werden. Beispiele für solche Reaktionen sind Additions- und Kondensationsreaktionen. Neben der radikalisch induzierten Vernetzung und dem Abkühlen findet somit nach Ablauf dieser Reaktionen auf eine dritte Weise eine mechanische Festigung des Gegenstandes statt.

In einer weiteren bevorzugten Ausführungsform enthält das radikalisch vernetzbare Aufbaumaterial eine Polyaminkomponente. In einem erwärmten Aufbaumaterial kann so (gegebenenfalls in Gegenwart von Urethanisierungskatalysatoren und/oder Umesterungskatalysatoren) eine chemische Reaktion im Aufbaumaterial stattfinden. Die durch Addition gebildeten Urethangruppen können sich unter diesen Bedingungen, wenigstens teilweise, reversibel öffnen, wodurch freie NCO-Gruppen zur Reaktion mit den Aminogruppen der Polyaminkomponente unter Bildung von Harnstoffgruppen zur Verfügung stehen. Auf diese Weise kann das durchschnittliche Molekulargewicht des Polyurethans erhöht werden. Im Fall von Polyaminen mit einer durchschnittlichen Funktionalität von > 2 kann weiterhin die Vernetzungsdichte im Aufbaumaterial erhöht werden. Wenn das Polyurethan wenigstens teilweise aus Polyesterpolyolen aufgebaut ist, kann als weitere Reaktion auch die Öffnung der Esterbindungen und Reaktion mit Polyaminen zu Amiden ablaufen. Auch hierdurch kann bevorzugt eine Nachhärtung des Aufbaumaterials nach Erhalt des Vorläufers erreicht werden. Das Gleiche gilt, wenn das Aufbaumaterial ein Polyesterpolymer umfasst.

Erfindungsgemäß einsetzbare Katalysatoren sind dabei solche, die die Urethanisierungsreaktion sowie auch die Rückreaktion zu Isocyanaten und Polyolen und/oder eine Amidierung der Urethanbindung zu Harnstoffen beschleunigen, und/oder solche, die eine Amidierung von Estergruppen zu Amiden beschleunigen. Geeignete Katalysatoren dafür sind dem Fachmann bekannt und können sowohl unter sauren, basischem als auch neutralem pH Bedingungen wirksam sein. In einer bevorzugten Variante wird die Umamidierungsreaktion ohne Zugabe von zusätzlichen Katalysatoren durchgeführt.

Geeignete Amine für die Polyaminkomponente sind insbesondere aliphatische Polyamine mit einer durchschnittlichen Aminfunktionalität von ≥ 2, vorzugsweise symmetrische aliphatische Polyamine mit einer durchschnittlichen Aminfunktionalität von ≥ 2 (lineare oder alizyklische Polyamine mit festen Geometrien bezüglich ihrer *cis-* oder trans-Konfigurationen).

In einer weiteren bevorzugten Ausführungsform weist die Polyaminkomponente eine durchschnittliche Anzahl von Zerewitinoff-aktiven H-Atomen von ≥ 4 auf.

In einer weiteren bevorzugten Ausführungsform enthält die Polyaminkomponente eine oder mehrere Verbindungen aus der Gruppe: Adipinsäuredihydrazid, Ethylendiamin, Diethylentriamin, Triethylentetramin, Tetraetylenpentamin, Pentaethylenhexamin, Dipropylentriamin, Hexamethylendiamin, Hydrazin, Isophorondiamin, (4,4'- und/oder 2,4'-)Diaminodicyclohexylmethan, (4,4'- und/oder 2,4'-)Diamino-3,3'-dimethyldicyclohexylmethan und N-(2-Aminoethyl-)2-aminoethanol.

In einer weiteren bevorzugten Ausführungsform liegt die Polyaminkomponente in einem Anteil von ≥ 0.1 Gewichts-% bis ≤ 25 Gewichts-%, bezogen auf das Gewicht des Aufbaumaterials, vor.

In einer weiteren bevorzugten Ausführungsform enthält das radikalisch vernetzbare Aufbaumaterial blockierte oder unblockierte NCO-Gruppen. Wenn die NCO-Gruppen blockiert sind, umfasst das erfindungsgemäße Verfahren weiterhin den Schritt des Deblockierens dieser NCO-Gruppen. Nach ihrer Deblockierung stehen sie so für weitere Reaktionen zur Verfügung.

Das Blockierungsmittel wird so gewählt, dass bei Erwärmung im erfindungsgemäßen Verfahren die NCO-Gruppen wenigstens teilweise deblockieren. Beispiele für Blockierungsmittel sind Alkohole, Lactame, Oxime, Malonester, Alkylacetoacetate, Triazole, Phenole, Imidazole, Pyrazole sowie Amine, wie z.B. Butanonoxim, Diisopropylamin, 1,2,4-Triazol, Dimethyl-1,2,4-triazol, Imidazol, Malonsäurediethylester, Acetessigester, Acetonoxim, 3,5-Dimethylpyrazol, ε-Caprolactam, N-Methyl-, N-Ethyl-, N-(Iso)propyl-, N-n-Butyl-, N-iso-Butyl-, N-tert.-Butyl-benzylamin oder 1,1-Dimethylbenzylamin, N-Alkyl-N-1,1- Dimethylmethylphenylamin, Addukte von Benzylamin an Verbindungen mit aktivierten Doppelbindungen wie Malonsäureestern, N,N-Dimethylaminopropylbenzylamin und andere tertiäre Aminogruppen enthaltende gegebenenfalls substituierte Benzylamine und/oder Dibenzylamin oder beliebige Gemische dieser Blockierungsmittel.

In einer weiteren bevorzugten Ausführungsform enthält das radikalisch vernetzbare Aufbaumaterial einen Isocyanat-Trimerisierungskatalysator. Vorzugsweise ist der Trimerisierungskatalysator Kaliumacetat, Kaliumacetat in Kombination mit einem Kronenether, Kaliumacetat in Kombination mit einem Polyethylenglykol, Kaliumacetat in Kombination mit einem Polypropylenglykol, Zinnoctoat, Natriumphenolat, Kaliumhydroxid, Trioctylphosphin, Tributylzinnoxid oder eine Mischung aus mindestens zwei der vorgenannten Katalysatoren. Die Reaktion zur Erhöhung der mechanischen Festigkeit ist somit die Bildung von Isocyanuraten aus freien NCO-Gruppen. Hierdurch kommt es zu einer weiteren Vernetzung des Aufbaumaterials.

In einer weiteren bevorzugten Ausführungsform enthält das radikalisch vernetzbare Aufbaumaterial Gruppen mit Zerewitinoff-aktiven H-Atomen und eine oder mehrere zyklische Zinnverbindungen der Formel F-1, F-II und/oder F-III: 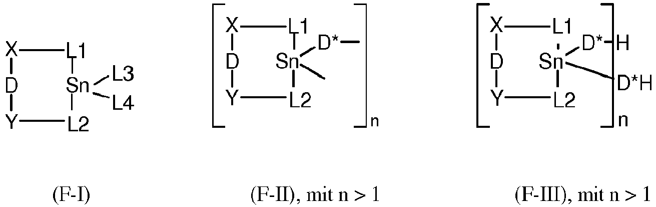 wobei gilt:
D steht für -O-, -S- oder -N(R1)-
   wobei R1 für einen gesättigten oder ungesättigten, linearen oder verzweigten, aliphatischen oder cycloaliphatischen oder einen gegebenenfalls substituierten, aromatischen oder araliphatischen Rest mit bis zu 20 Kohlenstoffatomen steht, der gegebenenfalls Heteroatome aus der Reihe Sauerstoff, Schwefel, Stickstoff enthalten kann, oder für Wasserstoff oder den Rest 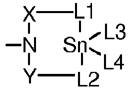 steht,
   oder R1 und L3 zusammen für -Z-L5- stehen;
D* steht für -O- oder -S-;
X, Y und Z stehen für gleiche oder unterschiedliche Reste ausgewählt aus Alkylenresten der Formeln -C(R2)(R3)-, -C(R2)(R3)-C(R4)(R5)- oder -C(R2)(R3)-C(R4)(RS)-C(R6)(R7)- oder ortho-Arylenresten der Formeln 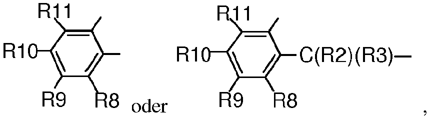 wobei R2 bis R11 unabhängig voneinander für gesättigte oder ungesättigte, lineare oder verzweigte, aliphatische oder cycloaliphatische oder gegebenenfalls substituierte, aromatische oder araliphatische Reste mit bis zu 20 Kohlenstoffatomen stehen, die gegebenenfalls Heteroatome aus der Reihe Sauerstoff, Schwefel, Stickstoff enthalten können, oder für Wasserstoff stehen;
L1, L2 und L5 stehen unabhängig voneinander für -O-, -S-, -OC(=O)-, -OC(=S)-, -SC(=O)-, - SC(=S)-, -OS(=O)₂O-, -OS(=O)₂- oder -N(R12)-,
   wobei R12 für einen gesättigten oder ungesättigten, linearen oder verzweigten, aliphatischen oder cycloaliphatischen oder einen gegebenenfalls substituierten, aromatischen oder araliphatischen Rest mit bis zu 20 Kohlenstoffatomen steht, der gegebenenfalls Heteroatome aus der Reihe Sauerstoff, Schwefel, Stickstoff enthalten kann, oder für Wasserstoff steht;
L3 und L4 stehen unabhängig voneinander für -OH, -SH, -OR13, -Hal, -OC(=O)R14, -SR15, - OC(=S)R16, -OS(=O)₂OR17, -OS(=O)₂R18 oder -NR19R20, oder L3 und L4 zusammen stehen für -L1-X-D-Y-L2-,
   wobei für R13 bis R20 unabhängig voneinander für gesättigte oder ungesättigte, lineare oder verzweigte, aliphatische oder cycloaliphatische oder gegebenenfalls substituierte, aromatische oder araliphatische Reste mit bis zu 20 Kohlenstoffatomen stehen, die gegebenenfalls Heteroatome aus der Reihe Sauerstoff, Schwefel, Stickstoff enthalten können, oder für Wasserstoff stehen.

Die Zinnverbindungen der Formeln F-I, F-II und F-III sind thermisch labil. Unterhalb einer bestimmten Temperatur weisen sie keine technisch sinnvolle katalytische Aktivität für die Reaktion von NCO-Gruppen mit funktionellen Gruppen, welche Zerewitinoff-aktive H-Atome tragen, auf. Insbesondere seien hierbei Urethanisierungen und Harnstoffbildungen zu nennen. Oberhalb einer bestimmten Temperatur steigt jedoch die katalytische Aktivität stark an. Ohne auf eine Theorie beschränkt zu sein wird angenommen, dass dann die Liganden Sn-Zentrum ganz oder teilweise dissoziieren und daher das Sn-Zentrum als Katalysator zur Verfügung steht. Insofern lässt sich von thermisch latenten Katalysatoren sprechen. Dadurch, dass die im Aufbaumaterial vorliegenden NCO-Gruppen unterhalb dieser Temperatur nicht abreagieren, kann das Aufbaumaterial auch leicht wiederverwertet werden.

In den Fällen, in denen die Zinnverbindungen der Formeln F-I, F-II und/oder F-III Liganden mit freien OH- und/oder NH-Resten aufweisen, kann der Katalysator bei der Polyisocyanat-Polyadditionsreaktion in das Produkt eingebaut werden. Besonderer Vorteil dieser einbaubaren Katalysatoren ist ihr stark reduziertes Fogging-Verhalten.

Die verschiedenen Herstellungsmethoden für die erfindungsgemäß zu verwendenden Zinn(IV)-Verbindungen bzw. ihrer Zinn(II)-Precursoren sind u.a. beschrieben in: J. Organomet. Chem. 2009 694 3184-3189, Chem. Heterocycl. Comp. 2007 43 813-834, Indian J. Chem. 1967 5 643-645 sowie in darin angeführter Literatur.

Der Gehalt der Zinnverbindungen der Formeln F-I, F-II und/oder F-III im Aufbaumaterial kann vom Typ der dem Aufbaumaterial zugrunde liegenden Isocyanate abhängig gemacht werden. So kann, wenn an ein aromatisches C-Atom gebundene NCO-Gruppen dominieren, der Gehalt ≤ 100 ppm, bezogen auf das Gesamtgewicht des Aufbaumaterials, betragen. Wenn an ein aliphatisches C-Atom gebundene NCO-Gruppen dominieren, der Gehalt ≤ 3000 ppm, bezogen auf das Gesamtgewicht des Aufbaumaterials, betragen.

Vorzugsweise liegen im Aufbaumaterial unblockierte NCO-Gruppen vor.

Als NCO-reaktive Verbindungen mit Zerewitinoff-aktiven H-Atomen können alle dem Fachmann bekannten Verbindungen eingesetzt werden, welche eine mittlere OH- bzw. NH-Funktionalität von mindestens 1,5 aufweisen. Dies können beispielsweise niedermolekulare Diole (z. B. 1,2-Ethandiol, 1,3- bzw. 1,2-Propandiol, 1,4-Butandiol, 1,5-Petandiol, 1,6-Hexandiol), Triole (z. B. Glycerin, Trimethylolpropan) und Tetraole (z. B. Pentaerythrit) sein, kurzkettige Aminoalkohole, Polyamine aber auch höhermolekulare Polyhydroxyverbindungen wie Polyetherpolyole, Polyesterpolyole, Polycarbonatpolyole, Polysiloxanpolyole, Polyamine und Polyetherpolyamine sowie Polybutadienpolyole.

In einer weiteren bevorzugten Ausführungsform werden als zyklische Zinnverbindung eine oder mehrere der nachfolgenden Verbindungen eingesetzt:
4,12-Di-n-butyl-1,7,9,15-tetraoxa-4,12-diaza-8-stannaspiro [7.7] pentadecan,
4,12-Di-n-butyl-2,6,10,14-tetramethyl-1,7,9,15-tetraoxa-4,12-diaza-8-stannaspiro[7.7]pentadecan,
2,4,6,10,12,14-Hexamethyl-1,7,9,15-tetraoxa-4,12-diaza-8-stannaspiro[7.7]pentadecan,
4,12-Di-n-octyl-2,6,10,14-tetramethyl-1,7,9,15-tetraoxa-4,12-diaza-8-stannaspiro[7.7]pentadecan,
4,12-Di-n-octyl-1,7,9,15-tetraoxa-4,12-diaza-8-stannaspiro[7.7]pentadecan,
4,12-Dimethyl-1,7,9,15-tetraoxa-4,12-diaza-8-stannaspixo[7.7]pentadecan,
1,1-Dichloro-5-methyl-5-aza-2,8-dioxa-1-stannacyclooctan,
1,1-Diisopropyl-5-methyl-5-aza-2,8-dioxa-1-stannacyclooctan,
1,1-Dibenzoyl-3,3,7,7-tetramethyl 5-n-octyl-5-aza-2,8-dioxa-1-stannacyclooctan,
1,1-Dibenzoyl- 5-n-octyl-5-aza-2,8-dioxa-1-stannacyclooctan,
1,1-Bis(p-dodecylphenylsulfonyl)- 5-n-octyl-5-aza-2,8-dioxa-1-stannacyclooctan,
2-Benzoyloxy-6-octyl-4,8-dioxo-1,3,6,2-dioxazastannocan-2-ylbenzoat
oder Mischungen davon.

In einer weiteren bevorzugten Ausführungsform enthält das radikalisch vernetzbare Aufbaumaterial Gruppen mit Zerewitinoff-aktiven H-Atomen und dass das Blockierungsmittel ein Isocyanat ist oder das Blockierungsmittel derart ausgewählt ist, dass nach Deblockierung der NCO-Gruppe keine Freisetzung des Blockierungsmittels als freies Molekül oder als Teil von anderen Molekülen oder Molekülteilen stattfindet. Dann können die nach der Deblockierung die erhaltenen funktionellen Gruppen mit Verbindungen mit mindestens zwei Zerewitinoff-aktiven H-Atomen reagieren, so eine Erhöhung der mechanischen Festigkeit oder eine Vernetzung eintritt. Insofern muss eine Deblockierung der NCO-Gruppen im Sinne der vorliegenden Erfindung nicht zwangsläufig bedeuten, dass wieder eine NCO-Gruppe erhalten wird. Vielmehr kann dieses auch bedeuten, dass eine funktionelle Gruppe wie eine Acylkation-Gruppe nach Deblockierung erhalten werden kann, welche mit anderen funktionellen Gruppen, die Zerewitinoff-aktive H-Atomen aufweisen, unter Ausbildung einer kovalenten Bindung reagiert.

Vorzugsweise wird die Reaktion durchgeführt, bis ≤ 50%, bevorzugt ≤ 30% und mehr bevorzugt ≤ 20% der ursprünglich in der härtbaren Komponente vorhandenen blockierten Isocyanat-Gruppen noch vorhanden sind. Dieses lässt sich mittels Oberflächen-IR-Spektroskopie bestimmen. Es ist weiter bevorzugt, dass nach Schritt IV) ≥ 50%, ≥ 60%, ≥ 70% oder ≥ 80%, der in der härtbaren Komponente deblockierten NCO-Gruppen mit der Verbindung mit mindestens zwei Zerewitinoff-aktiven H-Atomen reagieren.

Als NCO-reaktive Verbindungen mit Zerewitinoff-aktiven H-Atomen können auch hier alle dem Fachmann bekannten Verbindungen eingesetzt werden, welche eine mittlere OH- bzw. NH-Funktionalität von mindestens 1,5 aufweisen. Dies können beispielsweise niedermolekulare Diole (z. B. 1,2-Ethandiol, 1,3- bzw. 1,2-Propandiol, 1,4-Butandiol, 1,5-Petandiol, 1,6-Hexandiol), Triole (z. B. Glycerin, Trimethylolpropan) und Tetraole (z. B. Pentaerythrit) sein, kurzkettige Aminoalkohole, Polyamine aber auch höhermolekulare Polyhydroxyverbindungen wie Polyetherpolyole, Polyesterpolyole, Polycarbonatpolyole, Polysiloxanpolyole, Polyamine und Polyetherpolyamine sowie Polybutadienpolyole.

In einer bevorzugten Ausführungsform ist das Blockierungsmittel ausgewählt aus der Gruppe bestehend aus organischen Isocyanaten, Lactamen, Glycerincarbonat, einer Verbindung gemäß der allgemeinen Formel (I): 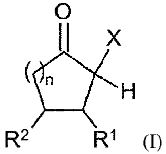 in welcher X eine elektronenziehende Gruppe, R¹ und R² unabhängig voneinander die Reste H, C₁-C₂₀-(Cyclo)alkyl, C₆-C₂₄-Aryl, C₁-C₂₀-(Cyclo)alkylester oder -amid, C₆-C₂₄-Arylester oder -amid, gemischt aliphatisch/aromatische Reste mit 1 bis 24 Kohlenstoffatomen, die auch Teil eines 4 bis 8-gliedrigen Ringes sein können, darstellen und n eine ganze Zahl von 0 bis 5 ist,
oder einer Kombination aus mindestens zwei hiervon.

Bei der elektronenziehenden Gruppe X kann es sich um alle Substituenten handeln, die zu einer CH-Azidität des α-ständigen Wasserstoffes führen. Dies können beispielsweise Estergruppen, Amidgruppen, Sulfoxidgruppen, Sulfongruppen, Nitrogruppen, Phosphonatgruppen, Nitrilgruppen, Isonitrilgruppen, Polyhalogenalkylgruppen, Halogene wie Fluor, Chlor oder Carbonylgruppen sein. Bevorzugt sind Nitril- und Estergruppen, besonders bevorzugt sind Carbonsäuremethylester- und Carbonsäureethylestergruppen. Geeignet sind auch Verbindungen der allgemeinen Formel (I), deren Ring gegebenenfalls Heteroatome, wie Sauerstoff-, Schwefel-, oder Stickstoffatome enthalten. Bevorzugt weist das aktivierte cyclische Keton der Formel (I) eine Ringgröße von 5 (n = 1) und 6 (n = 2) auf.

Bevorzugte Verbindungen der allgemeinen Formel (I) sind Cyclopentanon-2-carboxymethylester und -carboxyethylester, Cyclopentanon-2-carbonsäurenitril, Cyclohexanon-2-carboxymethylester und -carboxyethylester oder Cyclopentanon-2-carbonylmethyl. Besonders bevorzugt sind Cyclopentanon-2-carboxymethylester und -carboxyethylester sowie Cyclohexanon-2-carboxymethylester und-carboxyethylester. Die Cyclopentanonsysteme sind technisch leicht durch eine Dieckmann-Kondensation von Adipinsäuredimethylester oder Adipinsäurediethylester erhältlich. Cyclohexanon-2-carboxymethylester kann durch Hydrierung von Salicylsäuremethylester hergestellt werden.

Weitere bevorzugte Blockierungsmittel sind Glycerincarbonat, Lactame wie s-Caprolactam oder Isocyanate selbst. Die zu blockierende NCO-Gruppe kann unter Uretdionbildung mit der NCO-Gruppe des Blockierungsmittels reagieren. Die Rückreaktion führt wieder zur Bildung der NCO-Gruppen, welche mit den zur Verfügung stehenden Kettenverlängerern reagieren. Es ist besonders bevorzugt, wenn das Blockierungsmittel und die Verbindung mit der zu blockierenden NCO-Gruppe identisch sind. Dann beinhaltet die Blockierung eine Dimerisierung der betreffenden Verbindung. Dieses und die Reaktion mit Polyol und Polyamin ist in dem nachfolgenden Schema beispielhaft gezeigt.

In einer weiteren bevorzugten Ausführungsform belichtet und/oder bestrahlt Schritt II) eine Mehrzahl von Energiestrahlen den ausgewählten Bereich der Schicht des radikalisch vernetzbaren Aufbaumaterials, entsprechend dem jeweils ausgewählten Querschnitt des Vorläufers, gleichzeitig. Somit wird gemäß dieser Ausführungsform das additive Fertigungsverfahren der DLP-Technologie abgedeckt, wenn die Mehrzahl von Energiestrahlen über ein Array von einzeln ansteuerbaren Mikrospiegeln das per Belichtung und/oder Bestrahlung bereitzustellende Bild erzeugen.

In einer weiteren bevorzugten Ausführungsform werden in Schritt II) die abgeschiedenen Lagen des radikalisch vernetzten Aufbaumaterials auf eine Temperatur oberhalb des Schmelzpunktes des radikalisch vernetzbaren Polyurethans erwärmt. Die Temperatur kann ≥ 10 °C bis ≤ 100 °C, vorzugsweise ≥ 20 °C bis ≤ 80 °C und mehr bevorzugt ≥ 30 °C bis ≤ 60 °C über dem mittels DSC ermittelten Schmelzpunkt des radikalisch vernetzbaren Polyurethans liegen. Auf diese Weise können insbesondere die dritten Nachhärtungsmechanismen wie oben beschrieben schneller und/oder umfassender ablaufen.

In einer bevorzugten Ausführungsform des erfindungsgemä0en Verfahren, wird in Schritt IV) das Durchführen der chemischen Reaktion beinhaltet das Erwärmen auf eine Temperatur oberhalb des Schmelzpunktes des radikalisch vernetzbaren Polyurethans. Bevorzugt wird das Durchführen der chemischen Reaktion unter Erwärmen, oberhalb der Raumtemperatur RT von 23°C durchgeführt, bevorzugt bei einer Temperatur in einem Bereich von 50 bis 200 °C, oder bevorzugt in einem Bereich von 60 bis 180 °C, oder bevorzugt in einem Bereich von 80 bis 150 °C.

Ein weiterer Gegenstand der vorliegenden Erfindung ist ein System zur Herstellung eines Gegenstandes in einem additiven Fertigungsverfahren aus einem Vorläufer, umfassend einen entgegen der Schwerkraftrichtung anhebbaren Träger, eine Aufbaueinheit enthaltend ein geschmolzenes Aufbaumaterial unterhalb des Trägers und eine Bestrahlungs- und/oder Belichtungseinheit zum Bestrahlen und/oder Belichten von geschmolzenem Aufbaumaterial unterhalb des Trägers, wobei das Aufbaumaterial ein thermoplastisches radikalisch vernetzbares Polyurethan und einen Photoinitiator umfasst und dass das radikalisch vernetzbare Polyurethan einen Schmelzpunkt, bestimmt mittels dynamischer Differenzkalorimetrie, erste Aufheizung, bei einer Heizrate von 20 K/min, von ≥ 50 °C aufweist.

In einer bevorzugten Ausführungsform weist in dem System weist das radikalisch vernetzbare Polyurethan einen Schmelzpunkt von ≥ 60 °C bis ≤ 240 °C, mehr bevorzugt ≥ 80 °C bis ≤ 180 °C auf;
das radikalisch vernetzbare Polyurethan weist bei der Verarbeitungstemperatur eine Viskosität (DIN EN ISO 2884-1) von ≤ 10000 mPas (bevorzugt ≥ 50 mPas bis ≤ 8000 mPas, mehr bevorzugt ≥ 500 mPas bis ≤ 5000 mPas) auf;
der Photoinitiator bei der Verarbeitungstemperatur weist eine Halbwertszeit für seinen thermischen Abbau von ≥ 1 Stunde, oder bevorzugt ≥ 1,5 Stunden bis ≤ 5 Stunden, mehr bevorzugt ≥ 2 Stunden bis ≤ 4 Stunden; und/oder
in dem radikalisch vernetzbaren Aufbaumaterial liegen weitere, von radikalisch vernetzbaren funktionellen Gruppen verschiedene, funktionelle Gruppen in blockierter oder unblockierter Form vorliegen, welche in der Lage sind, eine von einer radikalischen Vernetzung verschiedene Reaktion zur Erhöhung der mechanischen Festigkeit im Aufbaumaterial einzugehen. Zu Details hinsichtlich dieser funktionellen Gruppen und ihrer Reaktionen sei zur Vermeidung von Wiederholungen auf die obigen Ausführungen zum erfindungsgemäßen Verfahren verwiesen.

Ein weiterer Gegenstand der vorliegenden Erfindung ist die Verwendung eines radikalisch vernetzbaren Aufbaumaterials in einem additiven Fertigungsverfahren bei einer Verarbeitungstemperatur von ≥ 50 °C, wobei das radikalisch vernetzbare Aufbaumaterial ein thermoplastisches radikalisch vernetzbares Polyurethan mit einem Urethangruppengehalt von ≥ 5 Gewichts-% und einem Schmelzpunkt, bestimmt mittels dynamischer Differenzkalorimetrie, erste Aufheizung, bei einer Heizrate von 20 K/min, von ≥ 50 ° C und einen Photoinitiator umfasst. Zu Details hinsichtlich des Aufbaumaterials einschließlich bevorzugter Varianten und Bereiche sei zur Vermeidung von Wiederholungen auf die obigen Ausführungen zum erfindungsgemäßen Verfahren verwiesen.

Es ist ebenfalls ein Polymer offenbart, erhältlich aus der radikalischen Vernetzung eines thermoplastischen radikalisch vernetzbaren Polyurethans mit einem Urethangruppengehalt von ≥ 5 Gewichts-%, wobei das radikalisch vernetzbare Polyurethan auf eine Verarbeitungstemperatur erwärmt wird, welche größer als der Schmelzpunkt des radikalisch vernetzbaren Polyurethans ist und wobei in dem radikalisch vernetzbaren Polyurethan weitere, von radikalisch vernetzbaren funktionellen Gruppen verschiedene, funktionelle Gruppen in blockierter oder unblockierter Form vorliegen, welche eine von einer radikalischen Vernetzung verschiedene Vernetzungsreaktion im Aufbaumaterial eingehen. Zu Details hinsichtlich des Aufbaumaterials einschließlich bevorzugter Varianten und Bereiche sei zur Vermeidung von Wiederholungen auf die obigen Ausführungen zum erfindungsgemäßen Verfahren verwiesen.

Schließlich ist eine Beschichtung offenbart, umfassend ein erfindungsgemäßes Polymer. Vorzugsweise ist die Beschichtung ein Lack oder ein Klebstoff.

Die vorliegende Erfindung wird anhand der nachfolgenden Figuren näher erläutert, ohne darauf beschränkt zu sein. Es zeigen:
- FIG. 1: ein erfindungsgemäßes System bei der Durchführung des erfindungsgemäßen Verfahrens
- FIG. 2: ein weiteres erfindungsgemäßes System bei der Durchführung des erfindungsgemäßen Verfahrens

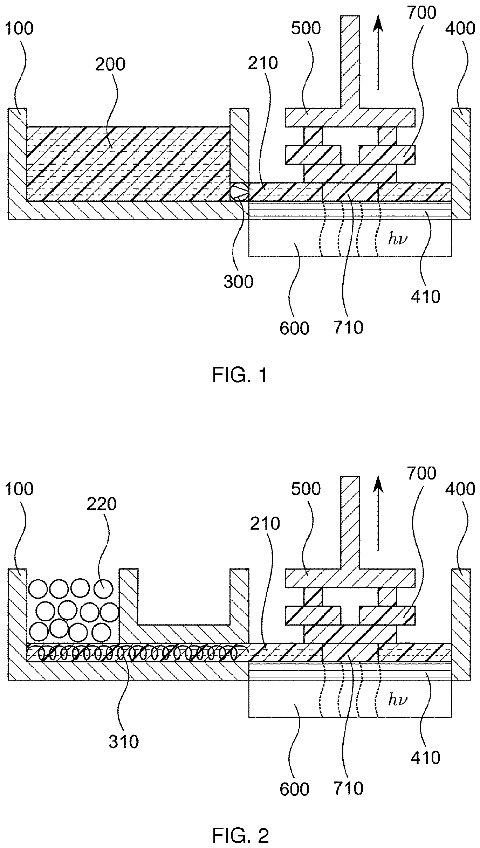

In dem in FIG. 1 dargestellten System stellt ein Vorratsbehälter 100 ein geschmolzenes Aufbaumaterial 200 bereit. Der Vorratsbehälter 100 ist zweckmäßigerweise thermisch isoliert und/oder weist eine geregelte Heizeinrichtung auf, um das geschmolzene Aufbaumaterial 200 auf eine vorbestimmte Temperatur zu temperieren. Über eine Pumpe 300 gelangt das geschmolzene Aufbaumaterial 200 zur Aufbaueinheit 400. Dort liegt das geschmolzene Aufbaumaterial in Form einer Schicht 210 vor, deren Dicke mit der Dicke einer Schicht des schichtweise aufzubauenden Vorläufers 700 korreliert. Es ist ebenfalls bevorzugt, dass die Aufbaueinheit 400 thermisch isoliert ist und/oder eine geregelte Heizeinrichtung aufweist, damit die Schicht 210 im geschmolzenen Zustand verbleibt. Der Boden der Aufbaueinheit 400 wird durch einen transparenten Boden 410, welcher beispielsweise aus Quarzglas gefertigt ist, gebildet. Durch einen Träger 500 wird der mit ihm verbundene aufzubauende Vorläufer 700 entgegen der Schwerkraftrichtung, in FIG. 1 durch den nach oben weisenden Pfeil symbolisiert, bewegt. Eine Belichtungseinheit 600 belichtet, entsprechend dem jeweiligen Querschnitt des aufzubauenden Vorläufers, die Schicht 210 des geschmolzenen Aufbaumaterials. Dieses ist in FIG. 1 durch "*hv*" symbolisiert.

Durch Einwirkung von Licht erfolgt in der Schicht 210 in einem vorbestimmten Bereich eine radikalische Vernetzung des geschmolzenen Aufbaumaterials unter Abscheidung einer Lage 710 auf eine zuvor abgeschiedene Lage des radikalisch vernetzten Aufbaumaterials. Anschließend wird der Träger 500 um eine vorbestimmte Strecke angehoben und mittels der Pumpe 300 die Schicht 210 vervollständigt. Auf diese Weise wird aus einzelnen Lagen der aufzubauende Vorläufer 700 erhalten. Da bereits gebildete Teile des Vorläufers 700 nicht mehr beheizt werden, sinkt deren Temperatur und es kommt durch Erstarrung zu einer weiteren mechanischen Verfestigung. In FIG. 1 ist nicht dargestellt, wie der Vorläufer 700 optional noch thermisch nachbehandelt wird, so dass es zu einer weiteren Verfestigung kommt.

In dem in FIG. 2 dargestellten System stellt ein Vorratsbehälter 100 ein Aufbaumaterial 220 in Granulatform bereit. Der Vorratsbehälter 100 ist kann thermisch isoliert sein und/oder eine geregelte Heizeinrichtung aufweisen, um das Aufbaumaterial 220 auf eine vorbestimmte Temperatur zu temperieren. Über eine beheizbare Extruderschnecke 310 gelangt das dadurch geschmolzene Aufbaumaterial zur Aufbaueinheit 400. Dort liegt das geschmolzene Aufbaumaterial in Form einer Schicht 210 vor, deren Dicke mit der Dicke einer Schicht des schichtweise aufzubauenden Vorläufers 700 korreliert. Es ist bevorzugt, dass die Aufbaueinheit 400 thermisch isoliert ist und/oder eine geregelte Heizeinrichtung aufweist, damit die Schicht 210 im geschmolzenen Zustand verbleibt. Der Boden der Aufbaueinheit 400 wird durch einen transparenten Boden 410, welcher beispielsweise aus Quarzglas gefertigt ist, gebildet. Durch einen Träger 500 wird der mit ihm verbundene aufzubauende Vorläufer 700 entgegen der Schwerkraftrichtung, in FIG. 2 durch den nach oben weisenden Pfeil symbolisiert, bewegt. Eine Belichtungseinheit 600 belichtet, entsprechend dem jeweiligen Querschnitt des aufzubauenden Vorläufers, die Schicht 210 des geschmolzenen Aufbaumaterials. Dieses ist in FIG. 1 durch *"hv"* symbolisiert.

Durch Einwirkung von Licht erfolgt in der Schicht 210 in einem vorbestimmten Bereich eine radikalische Vernetzung des geschmolzenen Aufbaumaterials unter Abscheidung einer Lage 710 auf eine zuvor abgeschiedene Lage des radikalisch vernetzten Aufbaumaterials. Anschließend wird der Träger 500 um eine vorbestimmte Strecke angehoben und mittels der Pumpe 300 die Schicht 210 vervollständigt. Auf diese Weise wird aus einzelnen Lagen der aufzubauende Vorläufer 700 erhalten. Da bereits gebildete Teile des Vorläufers 700 nicht mehr beheizt werden, sinkt deren Temperatur und es kommt durch Erstarrung zu einer weiteren mechanischen Verfestigung. In FIG. 2 ist nicht dargestellt, wie der Vorläufer 700 optional noch thermisch nachbehandelt wird, so dass es zu einer weiteren Verfestigung kommt.

### Beispiele

### Allgemeine Angaben:

Alle Prozentangaben beziehen sich, sofern nicht abweichend angegeben, auf Gewichtsprozent (Gew.-%).

Die zur Zeit der Versuchsdurchführung herrschende Umgebungstemperatur von 23 °C wird als RT (Raumtemperatur) bezeichnet. Wenn im Folgenden von Erwärmen gesprochen wird, bedeutet dies, dass das Aufbaumaterial auf eine Temperatur oberhalb von RT gebracht wird.

Die nachstehend aufgeführten Methoden zur Bestimmung der entsprechenden Parameter wurden zur Durchführung bzw. Auswertung der Beispiele angewendet und sind auch die Methoden zur Bestimmung der erfindungsgemäß relevanten Parameter im Allgemeinen.

### Bestimmung der Phasenübergänge mittels DSC

Die Phasenübergänge wurden mittels DSC (Differential Scanning Calorimetry) mit einem Mettler DSC 12E (Mettler Toledo GmbH, Gießen, DE) entsprechend DIN EN 61006 bestimmt. Eine Kalibrierung erfolgte durch die Temperatur des Schmelz-Onsets von Indium und Blei. Es wurden 10 mg Substanz in Normalkapseln eingewogen. Die Messung erfolgte durch zwei Aufheizungen von -50 °C bis +150 °C bei einer Heizrate von 20 K/min mit anschließender Abkühlung mit einer Kühlrate von 320 K/min. Die Kühlung erfolgte durch flüssigen Stickstoff. Als Spülgas wurde Stickstoff verwendet. Die angegebenen Werte basieren jeweils auf der Auswertung der 1. Aufheizkurve, da bei den untersuchten Reaktivsystemen durch die Temperaturbelastung in der DSC Veränderungen der Probe im Messprozess bei hohen Temperaturen möglich sind. Die Schmelztemperaturen Tₘ wurden aus den Temperaturen an den Maxima der Wärmeflusskurven erhalten. Die Glasübergangstemperatur T_{g} wurde aus der Temperatur bei der halben Höhe einer Glasübergangsstufe ermittelt.

### Bestimmung von Infrarotspektren

Die Infrarotspektren wurden an einem mit einer ATR-Einheit ausgerüstetem FT-IR-Spektrometer der Firma Bruker gemessen.

Für die relative Messung der Veränderung der freien NCO-Gruppen an Filmen wurde ein FT- IR-Spektrometer (Tensor II) der Fa. Bruker eingesetzt. Die Probe wurde mit der Platinum- ATR-Einheit kontaktiert. Die kontaktierte Fläche der Probe war 2 x 2 mm. Bei der Messung drang die IR-Strahlung je nach Wellenzahl 3- 4 µm in die Probe ein. Von der Probe wurde dann ein Absorptionsspektrum erstellt. Um eine ungleichmäßige Kontaktierung der unterschiedlich harten Proben zu kompensieren, wurde an allen Spektren eine Grundlinienkorrektur durchgeführt und eine Normierung im Wellenzahlbereich 2600-3200 (CH2, CH3). Die Interpolation der "freien" NCO-Gruppe wurde im Wellenzahlbereich 2170-2380 durchgeführt.

### Bestimmung der Viskosität

Sämtliche Viskositätsmessungen erfolgten mit einem Physica MCR 51 Rheometer der Fa. Anton Paar Germany GmbH (DE) nach DIN EN ISO 3219.

### Bestimmung des E-modul

Der E-Modul wurde an gehärteten Polymerfilmen im Zugversuch nach DIN 53371 ermittelt.

### Bestimmung der Härte

Die Mikrohärte wurde mittels eines Geräts Fischerscope H100C bestimmt und mittels DIN EN ISO 14577-1b anhand der Kraft-Eindringkurve die Martenshärte errechnet.

### Bestimmung der Topfzeit

Die Topfzeit wurde als Zeit bis zur Verdopplung der Viskosiät bei Verarbeitungstemperatur bestimmt.

### Bestimmung der Halbwertszeit des Photoinitiators

Die Halbwertszeit des Photoinitiators bei Verarbeitungstemperatur wurde als 10%tige Lösung in d8 Toluol bei Verarbeitungstemperatur bestimmt. Die Bestimmung des Abbaus erfolgte anhand Initiator spezifischer Signale des Produktspektrums mittels 1H bzw. 13C NMR Spektroskopie.

### Ausgangsverbindungen

Polyisocyanat A: HDI-Trimerisat (NCO-Funktionalität >3) mit einem NCO-Gehalt von 23,0 Gew.-% nach der Prüfmethode M105-ISO 11909, bezogen von der Fa. Covestro AG als Desmodur^{®} N3600. Die Viskosität beträgt ca. 1200 mPa·s bei 23°C (DIN EN ISO 3219/A.3).
Polyisocyanat B: HDI-Uretdion (NCO-Funktionalität >2) mit einem NCO-Gehalt von 21,8 Gew.-% nach der Prüfmethode M105-ISO 11909, bezogen von der Fa. Covestro AG als Desmodur^{®} N3400. Die Viskosität beträgt ca. 175 mPa·s bei 23°C (DIN EN ISO 3219/A.3).
Isophorondiisocyanat (IPDI), Funktionalität: 2, bezogen von der Fa. Covestro AG als Desmodur^{®} I.
Hexamethylendiisocyanat (HDI), Funktionalität: 2, bezogen von der Fa. Covestro AG als Desmodur^{®} H
Hydroxyethylmethacrylat (HEMA), bezogen von der Firma Sigma Aldrich.
Polytetrahydrofuran mit einem zahlenmittleren Molekulargewicht von ca. 1000 g/mol (PolyTHF1000), bezogen von der Firma Sigma Aldrich.
Ebecryl^{®} 4101, Urethanacrylat, Viskosität bei 25°C ca. 7000 mPas, zahlenmittleres Molekulargewicht ca. 1100 g/mol, Funktionalität ca. 3, bezogen von der Firma Allnex.
Ebecryl^{®} 4141, Urethanacrylat, Viskosität bei 25°C ca. 10000 mPas, zahlenmittleres Molekulargewicht ca. 700 g/mol, Funktionalität ca. 2, NCO-Gehalt ca. 12%, bezogen von der Firma Allnex.
Butylhydroxytoluol (BHT), bezogen von der Firma Sigma Aldrich.
2-(2-Dimethylaminoethoxy)ethanol, mit einer OH-Zahl von ca. 421 mg KOH/g, bezogen von der Firma Huntsman Corporation als Jeffcat^{®} ZR-70
Dibutylzinndilaurat (DBTL) bezogen von der Firma TIB Chemicals als TIB KAT 218.
2-Hydroxy-2-methylpropiophenon Photoinitiator, bezogen von der Firma IGM Resins als Omnirad^{®} 1173.

### Herstellung des verwendeten Trimerisierungskatalysators K:

Es wurde 2-(2-Dimethylaminoethoxy)ethanol mit einer OH-Zahl von 421 mg KOH/ mit einer bezogen auf die OH-Zahl äquimolaren Menge Isocyanat des Polyisocyanat A (ca. 23 Gew.% Isocyanat) durch Zutropfen unter Rühren und Kühlung bei Raumtemperatur umgesetzt bis kein OH mehr nachweisbar war und die Isocyanatkonzentration nach Titration < 0,5% war.

### Herstellung des nicht erfindungsgemäßen Urethanacrylat C1,

58,26 g des oligomeren Ausgangspolyisocyanats A1 wurden bei Raumtemperatur unter Rühren mit 41,67 g Hydroxyethylmethacrylat, sowie 0,05 g BHT und 0,02 g DBTL versetzt und anschließend für 3 h auf 50°C erwärmt und im Anschluss gerührt bis die Isocyanatkonzentration < 0,5% betrug.

Nach Abkühlen auf Raumtemperatur und weiterem Rühren für 5h lag ein klares hochviskoses Urethanacrylat mit einer Viskosität von 1250 mPas bei 75°C vor.

### Herstellung des erfindungsgemäßen Urethanacrylat C2*,

59,75 g des oligomeren Ausgangspolyisocyanats B wurden bei Raumtemperatur unter Rühren mit 40,18 g Hydroxyethylmethacrylat, sowie 0,05 g BHT und 0,02 g DBTL versetzt und anschließend für 3 h auf 50°C erwärmt und im Anschluss gerührt bis die Isocyanatkonzentration < 0,5% betrug. Nach Abkühlen auf Raumtemperatur lag ein bei 23°C festes Urethanacrylat mit einer Viskosität von 330 mPas bei 75°C vor. Urethangehalt: ca. 18%.

### Herstellung des erfindungsgemäßen Urethanacrylat C3*,

39,2 g von HDI werden bei Raumtemperatur unter Rühren mit 60,74 g Hydroxyethylmethacrylat, sowie 0,05 g BHT und 0,02 g DBTL versetzt und anschließend für 3 h auf 50°C erwärmt und im Anschluss gerührt bis die Isocyanatkonzentration < 0,5% beträgt. Nach Abkühlen auf Raumtemperatur liegt ein bei 23°C festes Urethanacrylat mit einer Viskosität von 63 mPas bei 75°C vor. Urethangehalt: ca. 27%.

### Herstellung der Formulierungen

Die radikalisch härtbaren Aufbaumaterialien gemäß den Beispielen 1 bis 6, wie in der nachfolgenden Tabelle aufgeführt, wurden in einem Glasgefäß abgewogen und auf eine Temperatur von 75°C gebracht und bei 75°C unter Rühren homogenisiert. Im Anschluss wurden die radikalisch härtbaren Aufbaumaterialien gemäß den Beispielen 1 bis 8 mit einem Rakel mit einem Spalt von 400 µm auf eine auf 80°C vorgewärmte Glasplatte aufgezogen.

Die beschichteten Glassubstrate wurden anschließend bei einer Harztemperatur von ca. 75°C in einer UV-Härtungsanlage der Firma Superfici mit Quecksilber- und Gallium-Strahlungsquellen mit einer Bandgeschwindigkeit von 2,5 m/min ausgehärtet. Aus Lampenleistung und Bandgeschwindigkeit resultiert eine Strahlungsintensität von 2600 mJ/cm², die auf die Beschichtungen in Form des Films einwirkte.

Anschließend wurden die UV-gehärteten Filme der Beispiele 6 bis 8 auf den Glassubstraten in einen Trockenofen bei 130 °C unter Luftatmosphäre für 1 Stunde gelagert.

Die Formulierungen der Beispiele 1 bis 6 und die Ergebnisse der Prüfungen an diesen und den daraus resultierenden gehärteten Filmen sind in den Tabellen 1 und 2 aufgelistet.

Erfindungsgemäße Beispiele sind mit einem * gekennzeichnet.

**Tabelle 1:**

| **Beispiel** | **1** | **2** | **3*** | **4*** |
|---|---|---|---|---|
| **Einsatzstoffe** | Gewicht [g] | | | |
| Ebecryl 4101 | 10 | | | |
| C1 | | 10 | | |
| C2* | | | 10 | |
| C3* | | | | 10 |
| Omnirad 1173 | 0,3 | 0,3 | 0,3 | 0,3 |

| **Ergebnisse der Prüfungen an den Formulierungen** | | | | |
|---|---|---|---|---|
| Bewertung bei Raumtemperatur | flüssig | flüssig | fest | fest |
| Viskosität bei Verarbeitungstemperatur von 75°C [mPas] | 250 | 1250 | 330 | 63 |
| T_{g} erste Aufheizung [°C] | -43,5 | -16,5 | -29 | -2,2 |
| Tₘ erste Aufheizung [°C] | - | - | 52,5 | 73,7 |

| **Ergebnisse der Prüfungen an den UV-gehärteten Formulierungen** | | | | |
|---|---|---|---|---|
| Bewertung Film | fester klarer Film | fester klarer Film | fester klarer Film | fester klarer Film |
| Martenshärte [N/mm²] | 5,95 | 97,67 | 86,91 | 186,13 |
| E-Modul (Zugversuch) [MPa] | 38 | 2060 | 1790 | 2680 |
| T_{g} erste Aufheizung [°C] | 1,5 | 122 | 109 | 123 |

**Tabelle 2:**

| **Beispiel** | **5*** | **6*** |
|---|---|---|
| **Einsatzstoffe** | Gewicht [g] | |
| C2* | 8 | |
| C3* | | 7 |
| Ebecryl^{®} 4141 | | 3 |
| PolyTHF1000 | 2 | |
| Trimerisierungskatalysators K | | 0,1 |
| DBTL | 0,002 | |
| Omnirad^{®} 1173 | 0,3 | 0,3 |

| **Ergebnisse der Prüfungen an den Formulierungen** | | |
|---|---|---|
| Topfzeit [h] | > 1 | > 1 |
| Lagerfähigkeit > 1 Monat unter Luft, keine sichtbaren Veränderungen | ok | ok |

| **Ergebnisse der Prüfungen an den UV-gehärteten Formulierungen** | | |
|---|---|---|
| Martenshärte (N/mm²) | 64,13 | 160,92 |
| IR: NCO Schwingung Peak Verhältnis zu CH2/CH3 Peak Schwingung | - | 0,52 |

| **Ergebnisse der Prüfungen an den UV-gehärteten Formulierungen nach Wärmelagerung** | | |
|---|---|---|
| Martenshärte (N/mm²) | 73,51 | 166,5 |
| IR: NCO Schwingung Peak Verhältnis zu CH2/CH3 Peak Schwingung | - | 0,13 |

Die erfindungsgemäßen Beispiele 3 bis 6 enthaltend bei Raumtemperatur feste trüb-weiße Urethanacrylate mit einem Schmelzpunkt oberhalb von Raumtemperatur. Sie zeichnen sich im Vergleich zu den nicht erfindungsgemäßen Beispielen 1 und 2 nach UV Härtung durch deutlich höhere Härten sowie E-Moduli, bezogen auf die Funktionalität der eingesetzten Isocyanate, und Erweichungspunkte bei gleichzeitig klaren transparenten Filmen aus.

Die erfindungsgemäßen Beispiele 5 und 6 zeigen die erfolgreiche Kombination bei Raumtemperatur nicht mischbarer reaktiver Zusammensetzungen durch eine erste UV Härtung oberhalb des Schmelzpunktes der festen Urethanacrylate mit anschließender Wärmehärtung der reaktiven Komponenten unter Ausbildung zusätzlicher Netzwerkpunkte und daraus resultierende Verbesserung der mechanischen Eigenschaften. Weiter zeigen die erfindungsgemäßen Beispiele bis 6, dass es möglich ist, unterhalb des Schmelzpunktes der Urethanacrylate metastabile Dualcure-Reaktivgemische herzustellen, die vor Gebrauch eine Lagerfähigkeit von > 1 Monat haben. Unter Lagerfähigkeit wird hier ein Viskositätserhöhung von maximal 50% verstanden

Durch den Einsatz der erfinderischen Formulierungen in UV härtenden und thermisch nachhärtenden Verfahren im 3D Druck, werden so zum ersten Mal Materialien ausgehend von schmelzbaren Dualcure Systemen mit hohem Urethangehalt und damit hoher mechanischer Verstärkung zugänglich, die außerdem lagerstabil sind.

## Patentansprüche

1. Verfahren zur Herstellung eines Gegenstandes in einem additiven Fertigungsverfahren aus einem Vorläufer (700), umfassend die Schritte:
I) Abscheiden einer Lage eines radikalisch vernetzten Aufbaumaterials, welche einem ersten ausgewählten Querschnitt des Vorläufers entspricht, auf einem Träger;
II) Abscheiden einer Lage eines radikalisch vernetzten Aufbaumaterials (710), welche einem weiteren ausgewählten Querschnitt des Vorläufers entspricht, auf eine zuvor aufgetragene Lage des radikalisch vernetzten Aufbaumaterials;
III) Wiederholen des Schritts II), bis der Vorläufer gebildet ist;
wobei das Abscheiden eines radikalisch vernetzten Aufbaumaterials (710) wenigstens in Schritt II) durch Belichten und/oder Bestrahlen eines ausgewählten Bereichs eines radikalisch vernetzbaren Aufbaumaterials, entsprechend dem jeweils ausgewählten Querschnitt des Vorläufers, erfolgt;
wobei der Träger (500) innerhalb eines Behälters angeordnet ist, der Behälter das radikalisch vernetzbare Aufbaumaterial bereitstellt, der Träger (500) vertikal entgegen der Schwerkraftrichtung anhebbar ist und weiterhin vor jedem Schritt II) der Träger (500) um eine vorbestimmte Strecke angehoben wird, so dass unter der in vertikaler Richtung gesehen untersten Lage des Aufbaumaterials sich eine Schicht des radikalisch vernetzbaren Aufbaumaterials bildet;
**dadurch gekennzeichnet, dass**
das radikalisch vernetzbare Aufbaumaterial ein thermoplastisches radikalisch vernetzbares Polyurethan mit einem Urethangruppengehalt von ≥ 5 Gewichts-% und einen Photoinitiator umfasst;
das radikalisch vernetzbare Aufbaumaterial in Schritt II) auf eine Verarbeitungstemperatur erwärmt wird, welche größer als der Schmelzpunkt, bestimmt mittels dynamischer Differenzkalorimetrie, erste Aufheizung, bei einer Heizrate von 20 K/min, des radikalisch vernetzbaren Polyurethans ist;
das radikalisch vernetzbare Polyurethan einen Schmelzpunkt, bestimmt mittels dynamischer Differenzkalorimetrie, erste Aufheizung, bei einer Heizrate von 20 K/min, von ≥ 50 °C aufweist;
und dass nach Schritt III) der eine Temperatur von 20 °C aufweisende Vorläufer als der Gegenstand definiert wird oder dass nach Schritt III) der Schritt IV) durchgeführt wird:
IV) Durchführen einer chemischen Reaktion im nach Schritt III) erhaltenen Vorläufer, so dass der Gegenstand erhalten wird.

2. Verfahren gemäß Anspruch 1, **dadurch gekennzeichnet, dass** das radikalisch vernetzbare Polyurethan bei der Verarbeitungstemperatur eine Viskosität (DIN EN ISO 2884-1) von ≤ 10000 mPas aufweist und/oder
der Photoinitiator bei der Verarbeitungstemperatur eine Halbwertszeit für seinen thermischen Abbau von ≥ 1 Stunde aufweist.

3. Verfahren gemäß Anspruch 1 oder 2, **dadurch gekennzeichnet, dass** in dem radikalisch vernetzbaren Aufbaumaterial weitere, von radikalisch vernetzbaren funktionellen Gruppen verschiedene, funktionelle Gruppen in blockierter oder unblockierter Form vorliegen, welche in der Lage sind, eine von einer radikalischen Vernetzung verschiedene Reaktion zur Erhöhung der mechanischen Festigkeit im Aufbaumaterial einzugehen.

4. Verfahren gemäß Anspruch 3, **dadurch gekennzeichnet, dass** das radikalisch vernetzbare Aufbaumaterial eine Polyaminkomponente enthält.

5. Verfahren gemäß Anspruch 3, **dadurch gekennzeichnet, dass** das radikalisch vernetzbare Aufbaumaterial blockierte oder unblockierte NCO-Gruppen enthält.

6. Verfahren gemäß Anspruch 5, **dadurch gekennzeichnet, dass** das radikalisch vernetzbare Aufbaumaterial einen Isocyanat-Trimerisierungskatalysator enthält.

7. Verfahren gemäß Anspruch 5 oder 6, **dadurch gekennzeichnet, dass** das radikalisch vernetzbare Aufbaumaterial Gruppen mit Zerewitinoff-aktiven H-Atomen und eine oder mehrere zyklische Zinnverbindungen der Formel F-I, F-II und/oder F-III enthält: 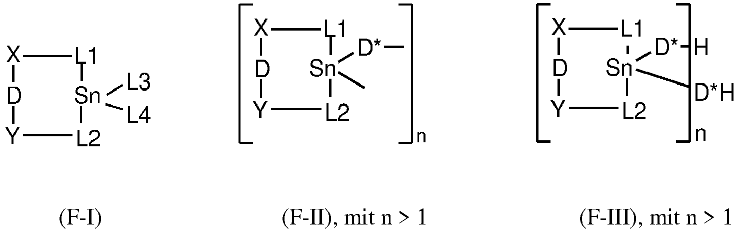 wobei gilt:
D steht für -O-, -S- oder -N(R1)-
wobei R¹ für einen gesättigten oder ungesättigten, linearen oder verzweigten, aliphatischen oder cycloaliphatischen oder einen gegebenenfalls substituierten, aromatischen oder araliphatischen Rest mit bis zu 20 Kohlenstoffatomen steht, der gegebenenfalls Heteroatome aus der Reihe Sauerstoff, Schwefel, Stickstoff enthalten kann, oder für Wasserstoff oder den Rest 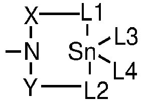 steht,
oder R¹ und L3 zusammen für -Z-L5- stehen;
D* steht für -O- oder -S-;
X, Y und Z stehen für gleiche oder unterschiedliche Reste ausgewählt aus Alkylenresten der Formeln -C(R2)(R3)-, -C(R2)(R3)-C(R4)(R5)- oder -C(R2)(R3)-C(R4)(RS)-C(R6)(R7)- oder ortho-Arylenresten der Formeln 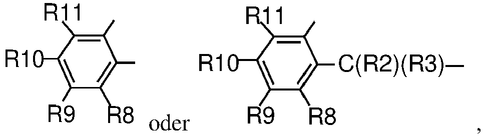 wobei R2 bis R11 unabhängig voneinander für gesättigte oder ungesättigte, lineare oder verzweigte, aliphatische oder cycloaliphatische oder gegebenenfalls substituierte, aromatische oder araliphatische Reste mit bis zu 20 Kohlenstoffatomen stehen, die gegebenenfalls Heteroatome aus der Reihe Sauerstoff, Schwefel, Stickstoff enthalten können, oder für Wasserstoff stehen;
L1, L2 und L5 stehen unabhängig voneinander für -O-, -S-, -OC(=O)-, -OC(=S)-, -SC(=O)-, - SC(=S)-, -OS(=O)₂O-, -OS(=O)₂- oder -N(R12)-,
wobei R12 für einen gesättigten oder ungesättigten, linearen oder verzweigten, aliphatischen oder cycloaliphatischen oder einen gegebenenfalls substituierten, aromatischen oder araliphatischen Rest mit bis zu 20 Kohlenstoffatomen steht, der gegebenenfalls Heteroatome aus der Reihe Sauerstoff, Schwefel, Stickstoff enthalten kann, oder für Wasserstoff steht;
L3 und L4 stehen unabhängig voneinander für -OH, -SH, -OR13, -Hal, -OC(=O)R14, -SR15, - OC(=S)R16, -OS(=O)₂OR17, -OS(=O)₂R18 oder -NR19R20, oder L3 und L4 zusammen stehen für -L1-X-D-Y-L2-,
wobei für R13 bis R20 unabhängig voneinander für gesättigte oder ungesättigte, lineare oder verzweigte, aliphatische oder cycloaliphatische oder gegebenenfalls substituierte, aromatische oder araliphatische Reste mit bis zu 20 Kohlenstoffatomen stehen, die gegebenenfalls Heteroatome aus der Reihe Sauerstoff, Schwefel, Stickstoff enthalten können, oder für Wasserstoff stehen.

8. Verfahren gemäß einem der Ansprüche 5 bis 7, **dadurch gekennzeichnet**, das radikalisch vernetzbare Aufbaumaterial Gruppen mit Zerewitinoff-aktiven H-Atomen enthält und dass das Blockierungsmittel ein Isocyanat ist oder das Blockierungsmittel derart ausgewählt ist, dass nach Deblockierung der NCO-Gruppe keine Freisetzung des Blockierungsmittels als freies Molekül oder als Teil von anderen Molekülen oder Molekülteilen stattfindet.

9. Verfahren gemäß einem der Ansprüche 1 bis 8, **dadurch gekennzeichnet, dass** in Schritt II) eine Mehrzahl von Energiestrahlen den ausgewählten Bereich der Schicht des radikalisch vernetzbaren Aufbaumaterials, entsprechend dem jeweils ausgewählten Querschnitt des Vorläufers, gleichzeitig belichtet und/oder bestrahlt.

10. Verfahren gemäß einem der Ansprüche 1 bis 9 **dadurch gekennzeichnet, dass** in Schritt IV) das Durchführen der chemischen Reaktion das Erwärmen auf eine Temperatur oberhalb des Schmelzpunktes des radikalisch vernetzbaren Polyurethans beinhaltet.

11. System zur Herstellung eines Gegenstandes in einem additiven Fertigungsverfahren aus einem Vorläufer, umfassend einen entgegen der Schwerkraftrichtung anhebbaren Träger (500), eine Aufbaueinheit (400) enthaltend ein geschmolzenes Aufbaumaterial (210) unterhalb des Trägers und eine Bestrahlungs- und/oder Belichtungseinheit (600) zum Bestrahlen und/oder Belichten von geschmolzenem Aufbaumaterial (210) unterhalb des Trägers (500), **dadurch gekennzeichnet, dass** das Aufbaumaterial ein thermoplastisches radikalisch vernetzbares Polyurethan und einen Photoinitiator umfasst und dass das radikalisch vernetzbare Polyurethan einen Schmelzpunkt, bestimmt mittels dynamischer Differenzkalorimetrie, erste Aufheizung, bei einer Heizrate von 20 K/min, von ≥ 50 °C aufweist.

12. System gemäß Anspruch 11, **dadurch gekennzeichnet, dass** das radikalisch vernetzbare Polyurethan bei der Verarbeitungstemperatur eine Viskosität (DIN EN ISO 2884-1) von ≤ 10000 mPas aufweist;
der Photoinitiator bei der Verarbeitungstemperatur eine Halbwertszeit für seinen thermischen Abbau von ≥ 1 Stunde aufweist und/oder
in dem radikalisch vernetzbaren Aufbaumaterial weitere, von radikalisch vernetzbaren funktionellen Gruppen verschiedene, funktionelle Gruppen in blockierter oder unblockierter Form vorliegen, welche in der Lage sind, eine von einer radikalischen Vernetzung verschiedene Reaktion zur Erhöhung der mechanischen Festigkeit im Aufbaumaterial einzugehen.

13. Verwendung eines radikalisch vernetzbaren Aufbaumaterials in einem additiven Fertigungsverfahren bei einer Verarbeitungstemperatur von ≥ 50 °C,
**dadurch gekennzeichnet, dass**
das radikalisch vernetzbare Aufbaumaterial ein thermoplastisches radikalisch vernetzbares Polyurethan mit einem Urethangruppengehalt von ≥ 5 Gewichts-% und einem Schmelzpunkt, bestimmt mittels dynamischer Differenzkalorimetrie, erste Aufheizung, bei einer Heizrate von 20 K/min, von ≥ 50 °C und einen Photoinitiator umfasst.

## Claims

1. Method of producing an article in an additive manufacturing method from a precursor (700), comprising the steps of:
I) depositing a layer of a free-radically crosslinked build material corresponding to a first selected cross section of the precursor on a carrier;
II) depositing a layer of a free-radically crosslinked build material (710) corresponding to a further selected cross section of the precursor onto a previously applied layer of the free-radically crosslinked build material;
III) repeating step II) until the precursor is formed; wherein the depositing of a free-radically crosslinked build material (710) at least in step II) is effected by exposure and/or irradiation of a selected region of a free-radically crosslinkable build material corresponding to the respectively selected cross section of the precursor;
wherein the carrier (500) is disposed within a container, the container provides the free-radically crosslinkable build material, the carrier (500) can be raised vertically counter to the direction of gravity and the carrier (500) is additionally raised by a predetermined distance prior to each step II), such that a layer of the free-radically crosslinkable build material forms below the lowermost layer of the build material as viewed in vertical direction;
**characterized in that**
the free-radically crosslinkable build material comprises a thermoplastic free-radically crosslinkable polyurethane having a urethane group content of ≥ 5% by weight and a photoinitiator;
the free-radically crosslinkable build material is heated in step II) to a processing temperature greater than the melting point, determined by dynamic differential calorimetry, first heating, at a heating rate of 20 K/min, of the free-radically crosslinkable polyurethane;
the free-radically crosslinkable polyurethane has a melting point, determined by dynamic differential calorimetry, first heating, at a heating rate of 20 K/min, of ≥ 50°C;
and **in that**, after step III), the precursor having a temperature of 20°C is defined as the article, or **in that**, after step III), step IV) is conducted:
IV) performing a chemical reaction in the precursor obtained after step III), such that the article is obtained.

2. Method according to Claim 1, **characterized in that** the free-radically crosslinkable polyurethane at the processing temperature has a viscosity (DIN EN ISO 2884-1) of ≤ 10 000 mPas; and/or
the photoinitiator at the processing temperature has a half-life for its thermal degradation of ≥ 1 hour.

3. Method according to Claim 1 or 2, **characterized in that** there are further functional groups in blocked or unblocked form other than free-radically crosslinkable functional groups in the free-radically crosslinkable build material, and these are capable of entering into a reaction other than free-radical crosslinking for increasing mechanical strength in the build material.

4. Method according to Claim 3, **characterized in that** the free-radically crosslinkable build material contains a polyamine component.

5. Method according to Claim 3, **characterized in that** the free-radically crosslinkable build material contains blocked or unblocked NCO groups.

6. Method according to Claim 5, **characterized in that** the free-radically crosslinkable build material contains an isocyanate trimerization catalyst.

7. Method according to Claim 5 or 6, **characterized in that** the free-radically crosslinkable build material contains groups having Zerewitinoff-active hydrogen atoms and one or more cyclic tin compounds of the formula F-I, F-II and/or F-III: 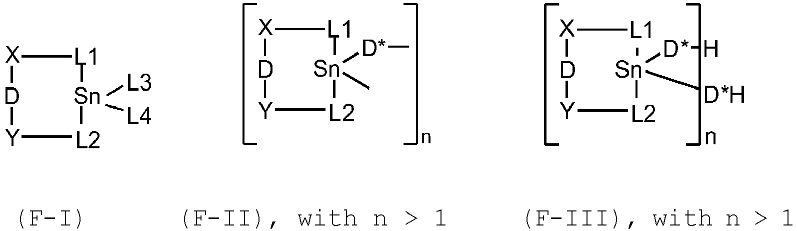 wherein:
D is -O-, -S- or -N(R1)-
where R1 is a saturated or unsaturated, linear or branched, aliphatic or cycloaliphatic radical or an optionally substituted aromatic or araliphatic radical which has up to 20 carbon atoms and may optionally contain heteroatoms from the group of oxygen, sulfur, nitrogen, or is hydrogen or the radical 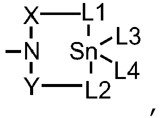
or R1 and L3 together are -Z-L5-;
D* is -O- or -S-;
X, Y and Z are identical or different radicals selected from alkylene radicals of the formulae -C(R2)(R3)-, -C(R2)(R3)-C(R4)(R5)- or -C(R2)(R3)-C(R4)(R5)-C(R6)(R7)- or ortho-arylene radicals of formulae 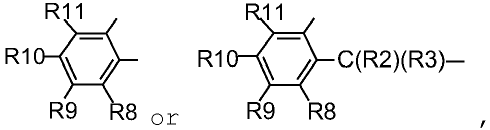 where R2 to R11 are independently saturated or unsaturated, linear or branched, aliphatic or cycloaliphatic or optionally substituted aromatic or araliphatic radicals which have up to 20 carbon atoms and may optionally contain heteroatoms from the group of oxygen, sulfur, nitrogen, or are hydrogen;
L1, L2 and L5 are independently -O-, -S-, -OC(=O)-, - OC(=S)-, -SC(=O)-, -SC(=S)-, -OS(=O)₂O-, -OS(=O)₂- or -N(R12)-,
where R12 is a saturated or unsaturated, linear or branched, aliphatic or cycloaliphatic radical or an optionally substituted aromatic or araliphatic radical which has up to 20 carbon atoms and may optionally contain heteroatoms from the group of oxygen, sulfur, nitrogen, or is hydrogen;
L3 and L4 are independently -OH, -SH, -OR13, -Hal, -OC(=O)R14, -SR15, -OC(=S)R16, -OS(=O)₂OR17, -OS(=O)₂R18 or -NR19R20, or L3 and L4 together represent -L1-X-D-Y-L2-,
where R13 to R20 are independently saturated or unsaturated, linear or branched, aliphatic or cycloaliphatic or optionally substituted aromatic or araliphatic radicals which have up to 20 carbon atoms and may optionally contain heteroatoms from the group of oxygen, sulfur, nitrogen, or are hydrogen.

8. Method according to any of Claims 5 to 7, **characterized in that** the free-radically crosslinkable build material contains groups having Zerewitinoff-active hydrogen atoms and **in that** the blocking agent is an isocyanate or the blocking agent is selected such that, after deblocking of the NCO group, no release of the blocking agent as a free molecule or as part of other molecules or molecular moieties takes place.

9. Method according to any of Claims 1 to 8, **characterized in that**, in step II), a multitude of energy beams simultaneously exposes and/or irradiates the selected region of the layer of the free-radically crosslinkable build material corresponding to the respectively selected cross section of the precursor.

10. Method according to any of Claims 1 to 9, **characterized in that**, in step IV), the performing of the chemical reaction includes heating to a temperature above the melting point of the free-radically crosslinkable polyurethane.

11. System for producing an article in an additive manufacturing method from a precursor, comprising a carrier (500) that can be raised counter to the direction of gravity, a build unit (400) comprising a molten build material (210) below the carrier and an irradiation and/or exposure unit (600) for irradiating and/or exposing molten build material (210) beneath the carrier (500), **characterized in that** the build material comprises a thermoplastic free-radically crosslinkable polyurethane and a photoinitiator, and **in that** the free-radically crosslinkable polyurethane has a melting point, determined by dynamic differential calorimetry, first heating, at a heating rate of 20 K/min, of ≥ 50°C.

12. System according to Claim 11, **characterized in that** the free-radically crosslinkable polyurethane at the processing temperature has a viscosity (DIN EN ISO 2884-1) of ≤ 10 000 mPas;
the photoinitiator at the processing temperature has a half-life for its thermal degradation of ≥ 1 hour and/or
there are further functional groups in blocked or unblocked form other than free-radically crosslinkable functional groups in the free-radically crosslinkable build material, and these are capable of entering into a reaction other than free-radical crosslinking for increasing mechanical strength in the build material.

13. Use of a free-radically crosslinkable build material in an additive manufacturing method at a processing temperature of ≥ 50°C,
**characterized in that**
the free-radically crosslinkable build material comprises a thermoplastic free-radically crosslinkable polyurethane having a urethane group content of ≥ 5% by weight and a melting point, determined by dynamic differential calorimetry, first heating, at a heating rate of 20 K/min, of ≥ 50°C, and a photoinitiator.

## Revendications

1. Procédé pour la fabrication d'un objet, dans un procédé de fabrication additive à partir d'un précurseur (700), comprenant les étapes :
I) dépôt d'une couche de matériau de construction réticulé par voie radicalaire, qui correspond à une première section transversale sélectionnée du précurseur, sur un support ;
II) dépôt d'une couche de matériau de construction réticulé par voie radicalaire (710), qui correspond à une autre section transversale sélectionnée du précurseur, sur une couche appliquée au préalable du matériau de construction réticulé par voie radicalaire ;
III) répétition de l'étape II) jusqu'à ce que le précurseur soit formé ;
le dépôt d'un matériau de construction réticulé par voie radicalaire (710) étant effectué, au moins dans l'étape II), par éclairage et/ou irradiation d'une zone sélectionnée d'un matériau de construction réticulable par voie radicalaire, correspondant à la section transversale à chaque fois sélectionnée du précurseur ;
le support (500) étant agencé dans un récipient, le récipient mettant à disposition le matériau de construction réticulable par voie radiculaire, le support (500) pouvant être soulevé verticalement à l'opposé du sens de la gravité et, en outre, avant chaque étape II),
le support (500) étant soulevé sur un trajet prédéterminé, de telle sorte qu'il se forme, sous la couche la plus basse vue dans la direction verticale du matériau de construction, une couche du matériau de construction réticulable par voie radicalaire ;
**caractérisé en ce que**
le matériau de construction réticulable par voie radicalaire comprend un polyuréthane thermoplastique réticulable par voie radicalaire présentant une teneur en groupes uréthane ≥ 5% en poids et un photo-initiateur ;
le matériau de construction réticulable par voie radicalaire dans l'étape II) est chauffé à une température de mise en oeuvre qui est supérieure au point de fusion, déterminé par calorimétrie différentielle dynamique, premier chauffage, à une vitesse de chauffage de 20 K/min, du polyuréthane réticulable par voie radicalaire ;
le polyuréthane réticulable par voie radicalaire présente un point de fusion, déterminé par calorimétrie différentielle dynamique, premier chauffage, à une vitesse de chauffage de 20 K/min, ≥ 50°C ;
et **en ce que**, après l'étape III), le précurseur présentant une température de 20°C est défini comme l'objet ou **en ce que** l'étape IV) est réalisée après l'étape III :
IV) réalisation d'une réaction chimique du précurseur obtenu après l'étape III), de telle sorte que l'objet est obtenu.

2. Procédé selon la revendication 1, **caractérisé en ce que** le polyuréthane réticulable par voie radicalaire présente, à la température de mise en oeuvre, une viscosité (DIN EN ISO 2884-1) ≤ 10.000 mPa.s et/ou le photo-initiateur présente, à la température de mise en oeuvre, une durée de demi-vie pour sa dégradation thermique ≥ 1 heure.

3. Procédé selon la revendication 1 ou 2, **caractérisé en ce que** le matériau de construction réticulable par voie radicalaire présente d'autres groupes fonctionnels, différents des groupes fonctionnels réticulables par voie radicalaire, sous forme bloquée ou non bloquée, qui sont en mesure de participer à une réaction différente d'une réticulation par voie radicalaire en vue d'augmenter la résistance mécanique dans le matériau de construction.

4. Procédé selon la revendication 3, **caractérisé en ce que** le matériau de construction réticulable par voie radicalaire contient un composant polyamine.

5. Procédé selon la revendication 3, **caractérisé en ce que** le matériau de construction réticulable par voie radicalaire contient des groupes NCO bloqués ou non bloqués.

6. Procédé selon la revendication 5, **caractérisé en ce que** le matériau de construction réticulable par voie radicalaire contient un catalyseur de trimérisation d'isocyanate.

7. Procédé selon la revendication 5 ou 6, **caractérisé en ce que** le matériau de construction réticulable par voie radicalaire contient des groupes présentant des atomes d'H actifs selon Zerewitinoff et un ou plusieurs composés cycliques de l'étain de formule F-I, F-II et/ou F-III : 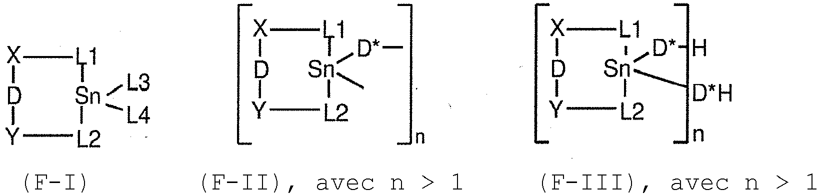 dans lesquelles :
D représente -O-, -S- ou -N(R1)-
R1 représentant un radical saturé ou insaturé, linéaire ou ramifié, aliphatique ou cycloaliphatique, ou un radical aromatique ou araliphatique le cas échéant substitué, comprenant jusqu'à 20 atomes de carbone, qui peut le cas échéant contenir des hétéroatomes de la série oxygène, soufre, azote, ou représentant hydrogène ou le radical 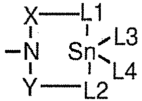
ou R1 et L3 représentant ensemble -Z-L5- ;
D* représente -O- ou -S- ;
X, Y et Z représentent des radicaux identiques ou différents choisis parmi les radicaux alkylène des formules -C(R2)(R3)-, -C(R2) (R3) -C(R4) (R5) - ou -C(R2) (R3) -C(R4) (R5) -C(R6) (R7) - ou des radicaux ortho-arylène des formules 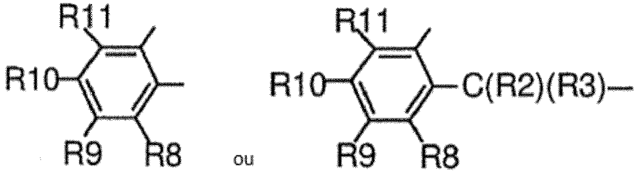
R2 à R11 représentant, indépendamment les uns des autres, des radicaux saturés ou insaturés, linéaires ou ramifiés, aliphatiques ou cycloaliphatiques, ou des radicaux aromatiques ou araliphatiques le cas échéant substitués, comprenant jusqu'à 20 atomes de carbone, qui peuvent le cas échéant contenir des hétéroatomes de la série oxygène, soufre, azote, ou représentant hydrogène ;
L1, L2 et L5 représentent indépendamment les uns des autres -O-, -S-, -OC(=O)-, -OC(=S)-, -SC(=O)-, -SC(=S)-, -OS(=O)₂O-, -OS(=O)₂- ou -N(R12)-,
R12 représentant un radical saturé ou insaturé, linéaire ou ramifié, aliphatique ou cycloaliphatique, ou un radical aromatique ou araliphatique le cas échéant substitué, comprenant jusqu'à 20 atomes de carbone, qui peut le cas échéant contenir des hétéroatomes de la série oxygène, soufre, azote, ou représentant hydrogène ;
L3 et L4 représentent, indépendamment l'un de l'autre, -OH, -SH, -OR13, -Hal, -OC(=O)R14, -SR15, -OC( =S)R16, -OS(=O)₂OR17, -OS(=O)₂R18 ou -NR19R20, ou L3 et L4 représentent ensemble -L1-X-D-Y-L2-,
R13 à R20 représentant, indépendamment les uns des autres, des radicaux saturés ou insaturés, linéaires ou ramifiés, aliphatiques ou cycloaliphatiques, ou des radicaux aromatiques ou araliphatiques le cas échéant substitués, comprenant jusqu'à 20 atomes de carbone, qui peuvent le cas échéant contenir des hétéroatomes de la série oxygène, soufre, azote, ou représentant hydrogène,

8. Procédé selon l'une des revendications 5 à 7, **caractérisé en ce que** le matériau de construction réticulable par voie radicalaire contient des groupes présentant des atomes d'H actifs selon Zerewitinoff et **en ce que** l'agent de blocage est un isocyanate ou l'agent de blocage est choisi de manière telle qu'après un déblocage du groupe NCO aucune libération de l'agent de blocage en tant que molécule libre ou en tant que partie d'autres molécules ou de parties moléculaires ne se produit.

9. Procédé selon l'une des revendications 1 à 8, **caractérisé en ce que** dans l'étape II), une multitude de rayons énergétiques éclairent et/ou irradient simultanément la zone sélectionnée de la couche de matériau de construction réticulable par voie radicalaire, conformément à la section transversale respective sélectionnée du précurseur.

10. Procédé selon l'une des revendications 1 à 9, **caractérisé en ce que** dans l'étape IV), la réalisation de la réaction chimique comporte le chauffage à une température supérieure au point de fusion du polyuréthane réticulable par voie radicalaire.

11. Système pour la fabrication d'un objet dans un procédé de fabrication additive à partir d'un précurseur, comprenant un support (500) pouvant être soulevé à l'opposé du sens de la gravité, une unité de construction (400) contenant un matériau de construction (210) fondu sous le support et une unité d'irradiation et/ou d'éclairage (600) destinée à irradier et/ou à éclairer le matériau de construction (210) fondu sous le support (500), **caractérisé en ce que** le matériau de construction comprend un polyuréthane thermoplastique réticulable par voie radicalaire et un photo-initiateur et **en ce que** le polyuréthane réticulable par voie radicalaire présente un point de fusion, déterminé par calorimétrie différentielle dynamique, premier chauffage, à une vitesse de chauffage de 20 K/min, ≥ 50°C.

12. Système selon la revendication 11, **caractérisé en ce que** le polyuréthane réticulable par voie radicalaire présente, à la température de mise en oeuvre, une viscosité (DIN EN ISO 2884-1) ≤ 10.000 mPa.s ;
le photo-initiateur présente, à la température de mise en oeuvre, une durée de demi-vie pour sa dégradation thermique ≥ 1 heure et/ou
le matériau de construction réticulable par voie radicalaire présente d'autres groupes fonctionnels, différents des groupes fonctionnels réticulables par voie radicalaire, sous forme bloquée ou non bloquée, qui sont en mesure de participer à une réaction différente d'une réticulation par voie radicalaire en vue d'augmenter la résistance mécanique dans le matériau de construction.

13. Utilisation d'un matériau de construction réticulable par voie radicalaire dans un procédé de fabrication additive à une température de mise en oeuvre ≥ 50°C,
**caractérisée en ce que**
le matériau de construction réticulable par voie radicalaire comprend un polyuréthane thermoplastique réticulable par voie radicalaire présentant une teneur en groupes uréthane ≥ 5% en poids et un point de fusion, déterminé par calorimétrie différentielle dynamique, premier chauffage, à une vitesse de chauffage de 20 K/min, ≥ 50°C et un photo-initiateur.
